(19) Europäisches Patentamt
European Patent Office
Office européen des brevets

(11) EP 1 280 897 B1

# (12) EUROPEAN PATENT SPECIFICATION

(45) Date of publication and mention of the grant of the patent:
**20.09.2006 Bulletin 2006/38**

(21) Application number: **01927920.7**

(22) Date of filing: **25.04.2001**

(51) Int Cl.:
***C12N 15/12*** (2006.01) ***C12N 15/62*** (2006.01)
***C12N 5/10*** (2006.01) ***C07K 14/47*** (2006.01)
***C12Q 1/68*** (2006.01) ***G01N 33/50*** (2006.01)
***G01N 33/68*** (2006.01) ***A61K 38/17*** (2006.01)
***A61K 31/7105*** (2006.01) ***A61K 31/713*** (2006.01)
***A61P 11/06*** (2006.01)

(86) International application number:
**PCT/EP2001/004635**

(87) International publication number:
**WO 2001/081574 (01.11.2001 Gazette 2001/44)**

## (54) REGULATION OF NF-AT INTERACTING PROTEIN NIP 45 VARIANT

REGULATION EINER NF-AT INTERAGIERENDEN PROTEINVARIANTE NIP 45

REGULATION DE VARIANTE DE PROTEINE NIP 45 A INTERACTION AVEC NF-AT

(84) Designated Contracting States:
**AT BE CH CY DE DK ES FI FR GB GR IE IT LI LU MC NL PT SE TR**

(30) Priority: **25.04.2000 US 199356 P**

(43) Date of publication of application:
**05.02.2003 Bulletin 2003/06**

(73) Proprietor: **Bayer HealthCare AG**
**51368 Leverkusen (DE)**

(72) Inventor: **ENCINAS, Jeffrey**
**Nara,**
**Nara 631-0032 (JP)**

(56) References cited:
**WO-A-97/39721** **WO-A-99/21993**

- **HODGE M R ET AL: "NF-AT-DRIVEN INTERLEUKIN-4 TRANSCRIPTION POTENTIATED BY NIP45" SCIENCE, AMERICAN ASSOCIATION FOR THE ADVANCEMENT OF SCIENCE,, US, vol. 274, no. 5294, 13 December 1996 (1996-12-13), page 1903 XP002045448 ISSN: 0036-8075 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

<110> Bayer AG

<120> REGULATION OF NF-AT INTERACTING PROTEIN NIP 45 VARIANT

<130> RCK-2 Foreign Countries

<140>
<141>

<150> US 60/199,356
<151> 2000-04-25

<160> 16

<170> PatentIn Ver. 2.1

<210> 1
<211> 2232
<212> DNA
<213> Homo sapiens

<400> 1

```
ggaaagtgtg ccatggcgga gcctgtgggg aagcggggcc gctggtccgg aggtagcggt 60
gccggccgag ggggtcgggg cggctggggc ggtcggggcc gggctcctcg ggcccagcgg 120
tctccatccc ggggcacgct ggacgtagtg tctgtggact tggtcaccga cagcgatgag 180
gaaattctgg aggtcgccac cgctcgcggt gccgcggacg aggttgaggt ggagcccccg 240
gagcccccgg ggccggtcgc gtcccgggat aacagcaaca gtgacagcga aggggaggac 300
aggcggcccg caggaccccc gcgggagccg gtcaggcggc ggcggcggct ggtgctggat 360
ccgggggagg cgccgctggt tccggtgtac tcggggaagg ttaaaagcag ccttcgcctt 420
atcccagatg atctatccct cctgaaactc taccctccag ggatgagga agaggcagag 480
ctggcagatt cgagtggtct ctaccatgag ggctccccat caccaggctc tccctggaag 540
acaaagctga ggactaagga taaagaagag aagaaaaaga cagagtttct ggatctggac 600
aactctcctc tgtccccacc ttcaccaagg accaaaagca gaacgcatac tcgggcactc 660
aagaagttaa gtgaggtgaa caagcgcctc caggatctcc gttcctgtct gagccccaag 720
ccacctcagg gtcaagagca acagggccaa gaggatgaag tggtcttggt ggaagggccc 780
accctcccag agacccccg actcttccca ctcaaaatcc gttgccgggc tgacctggtc 840
agattgcccc tcaggatgga ttcccctcta aagaccctca tgtcccacta tgaggaggcc 900
atgggactgt cgggacggaa gctctccttc ttctttgatg ggacaaagct ttcaggcagg 960
gagctgccag ctgacctggg catggaatct ggggacctca ttgaggtctg ggctgacacc 1020
ccactccctg tttgacggcc cagcctggac ttggggagaa tgactttccc ttttttgccc 1080
cataagggct agcataagct gaggtagaac ttatctttaa gctgcagcaa aatcaaggag 1140
tgacttttgt cccctctcct gttgaccctg gtttagagcc gttaaccact tggtgagtta 1200
tgtgggtgtt gttgccctgg gtggcctgtg gctccgtcca caagtcatgc tgagttttgc 1260
agcctctgtg acttggagat gtcccttcac cctcccctt tcaccaccat cctcttttcc 1320
tcatggaaat gtctgcttta tgaaactatg cacatattga aagtgagttg aaacaaatga 1380
gggttgggta ggagcttcca ggcctgggat ttacaccacg cctagcccag cagaggcctt 1440
```

```
agtcccattt ggggcttggg agtgacattt gcttgaggct tatacactgg tgtggttgcc 1500
tggcttgcag gaaatgacca agctcacaca tgctggctga agcgtaagca gacaactgag 1560
gtactctttt gaaggatgaa ggtggtggat tctcagccct gggggtcttc ctcacctgag 1620
gacccttcag agccaccctt tctagtttgc atttcctggt gcacacattt aaggcataac 1680
agcacattca tccctttggt ttgggatctc aggaatacag tcccatgcaa agattctctg 1740
gttttatggc ttttttccct ttctttacac catcctctcc cataagcacc catgtctttg 1800
aatatgaatg tatttgtaaa ataccacgtt tcatgtgtga atatgtgctt ttactgtaca 1860
tagtgctatt gtgcaatagg tcttatgctg ttttcactca atgtgtgcta agatctagcc 1920
ccattgactc ttctagaaat gcagtattgc tttgacctgc catgtggcac tccacaatgt 1980
caattgcagt ttacacacat tgcctaaagt gggggacacc tgggtgcccc tgaccccttg 2040
gcaccggata caggccacga taaacatcct ttcgtgtgtt cccttctgtg cttgtgtggc 2100
atgtgtaccc aggatgggcc tataggtcac agaggtcagt ttctctttgg ttttccagat 2160
tttctttaga acggtgactg accctcctac ttgaggccgc cctttctcc ttatccttgc 2220
cagcacttgt at                                                     2232
```

<210> 2
<211> 1005
<212> DNA
<213> Homo sapiens

<220>
<221> gene
<222> (1)..(1005)
<223> NIP45V1-ORF

<400> 2

```
atggcggagc ctgtggggaa gcggggccgc tggtccggag gtagcggtgc cggccgaggg 60
ggtcggggcg gctggggcgg tcggggccgg gctcctcggg cccagcggtc tccatcccgg 120
ggcacgctgg acgtagtgtc tgtggacttg gtcaccgaca gcgatgagga aattctggag 180
gtcgccaccg ctcgcggtgc cgcggacgag gttgaggtgg agcccccgga gcccccgggg 240
ccggtcgcgt cccgggataa cagcaacagt gacagcgaag gggaggacag gcggcccgca 300
ggacccccgc gggagccggt caggcggcgg cggcggctgg tgctggatcc gggggaggcg 360
ccgctggttc cggtgtactc ggggaaggtt aaaagcagcc ttcgccttat cccagatgat 420
ctatccctcc tgaaactcta ccctccaggg gatgaggaag aggcagagct ggcagattcg 480
agtggtctct accatgaggg ctccccatca ccaggctctc cctggaagac aaagctgagg 540
actaaggata aagaagagaa gaaaaagaca gagtttctgg atctggacaa ctctcctctg 600
tccccacctt caccaaggac caaaagcaga acgcatactc gggcactcaa gaagttaagt 660
gaggtgaaca agcgcctcca ggatctccgt tcctgtctga gccccaagcc acctcagggt 720
caagagcaac agggccaaga ggatgaagtg gtcttggtgg aagggcccac cctcccagag 780
accccccgac tcttcccact caaaatccgt tgccgggctg acctggtcag attgcccctc 840
aggatggatt cccctctaaa gaccctcatg tcccactatg aggaggccat gggactgtcg 900
ggacggaagc tctccttctt ctttgatggg acaaagcttt caggcaggga gctgccagct 960
gacctgggca tggaatctgg ggacctcatt gaggtctggg gctga                 1005
```

<210> 3
<211> 1115
<212> DNA
<213> Homo sapiens

<220>
<221> gene
<222> (1)..(1115)
<223> NIP45V2-ORF

<400> 3

```
atggcggagc ctgtggggaa gcggggccgc tggtccggag gtagcggtgc cggccgaggg 60
ggtcggggcg gctggggcgg tcggggccgg gctcctcggg cccagcggtc tccatcccgg 120
ggcacgctgg acgtagtgtc tgtggacttg gtcaccgaca gcgatgagga aattctggag 180
gtcgccaccg ctcgcggtgc cgcggacgag gttgaggtgg agcccccgga gcccccgggg 240
ccggtcgcgt cccgggataa cagcaacagt gacagcgaag gggaggacag gcggcccgca 300
ggacccccgc gggagccggt caggcggcgg cggcggctgg tgctggatcc gggggaggcg 360
ccgctggttc cggtgtactc ggggaaggtt aaaagcagcc ttcgccttat cccagatgat 420
ctatccctcc tgaaactcta ccctccaggg gatgaggaag aggcagagct ggcagattcg 480
agtggtctct accatgaggg ctccccatca ccaggctctc cctggaagac aaagctgagg 540
actaaggata aagaagagaa gaaaaagaca gagtttctgg atctggacaa ctctcctctg 600
tccccacctt caccaaggac caaaagcaga acgcatactc gggcactcaa gaagttaagt 660
gaggtgaaca agcgcctcca ggatctccgt tcctgtctga gccccaagcc acctcagggt 720
caagagcaac agggccaaga ggatgaagtg gtcttggtgg aagggcccac cctcccagag 780
acccccgac tcttcccact caaaatccgt tgccgggctg acctggtcag attgcccctc 840
aggatgactg tgtggtacta acaagttctc cagaggccac agagacgtcc caacagctcc 900
agctccgggt gcagggaaag gagaaacacc agacactgga agtctcactg tctcgagatt 960
cccctctaaa gaccctcatg tcccactatg aggaggccat gggactgtcg ggacggaagc 1020
tctccttctt ctttgatggg acaaagcttt caggcaggga gctgccagct gacctgggca 1080
tggaatctgg ggacctcatt gaggtctggg gctga                            1115
```

<210> 4
<211> 913
<212> DNA
<213> Homo sapiens

<220>
<221> gene
<222> (1)..(913)
<223> NIP45V3-ORF

<400> 4

```
atggcggagc ctgtggggaa gcggggccgc tggtccggag gttaaaagca gccttcgcct 60
tatcccagat gatctatccc tcctgaaact ctaccctcca ggggatgagg aagaggcaga 120
gctggcagat tcgagtggtc tctaccatga gggctcccca tcaccaggct ctccctggaa 180
gacaaagctg aggactaagg ataaagaaga gaagaaaaag acagagtttc tggatctgga 240
caactctcct ctgtccccac cttcaccaag gaccaaaagc agaacgcata ctcgggcact 300
caagaagtta agtgaggtga acaagcgcct ccaggatctc cgttcctgtc tgagccccaa 360
gccacctcag ggtcaagagc aacagggcca agaggatgaa gtggtcttgg tggaagggcc 420
```

caccctccca gagaccccccc gactcttccc actcaaaatc cgttgccggg ctgacctggt 480
cagattgccc ctcaggatgt cggagcccct gcagagtgtg gtggaccaca tggccaccca 540
ccttggggtg tccccaagca ggatcctttt gctttttgga gagacagagc tatcacctac 600
tgccactccc aggaccctaa agctcggagt ggctgacatc attgactgtg tggtactaac 660
aagttctcca gaggccacag agacgtccca acagctccag ctccgggtgc agggaaagga 720
gaaacaccag acactggaag tctcactgtc tcgagattcc cctctaaaga ccctcatgtc 780
ccactatgag gaggccatgg gactgtcggg acggaagctc tccttcttct ttgatgggac 840
aaagctttca ggcagggagc tgccagctga cctgggcatg gaatctgggg acctcattga 900
ggtctggggc tga 913

<210> 5
<211> 272
<212> DNA
<213> Homo sapiens

<220>
<221> gene
<222> (1)..(272)
<223> NIP45V4-ORF

<400> 5

atggcggagc ctgtggggaa gcggggccgc tggtccggag gttaaaagca gccttcgcct 60
tatcccagat gatctatccc tcctgaaact ctaccctcca ggggatgagg aaggattccc 120
ctctaaagac cctcatgtcc cactatgagg aggccatggg actgtcggga cggaagctct 180
ccttcttctt tgatgggaca aagctttcag gcagggagct gccagctgac ctgggcatgg 240
aatctgggga cctcattgag gtctggggct ga 272

<210> 6
<211> 2576
<212> DNA
<213> Homo sapiens

<220>
<221> gene
<222> (1)..(2576)
<223> NIP45 cDNA

<300>
<310> WO 99/21993
<311> 1998-10-21
<312> 1999-05-06

<400> 6

ggaaagtgtg ccatggcgga gcctgtgggg aagcggggcc gctggtccgg aggtagcggt 60
gccggccgag gggtcgggg cggctggggc ggtcggggcc gggctcctcg ggcccagcgg 120
tctccatccc ggggcacgct ggacgtagtg tctgtggact tggtcaccga cagcgatgag 180

```
gaaattctgg aggtcgccac cgctcgcggt gccgcggacg aggttgaggt ggagcccccg 240
gagcccccgg ggccggtcgc gtcccgggat aacagcaaca gtgacagcga aggggaggac 300
aggcggcccg caggaccccc gcgggagccg gtcaggcggc ggcggcggct ggtgctggat 360
ccgggggagg cgccgctggt tccggtgtac tcggggaagg ttaaaagcag ccttcgcctt 420
atcccagatg atctatccct cctgaaactc taccctccag ggatgagga agaggcagag 480
ctggcagatt cgagtggtct ctaccatgag ggctccccat caccaggctc tccctggaag 540
acaaagctga ggactaagga taaagaagag aagaaaaaga cagagtttct ggatctggac 600
aactctcctc tgtccccacc ttcaccaagg accaaaagca gaacgcatac tcgggcactc 660
aagaagttaa gtgaggtgaa caagcgcctc caggatctcc gttcctgtct gagccccaag 720
ccacctcagg gtcaagagca acagggccaa gaggatgaag tggtcttggt ggaagggccc 780
accctcccag agaccccccg actcttccca ctcaaaatcc gttgccgggc tgacctggtc 840
agattgcccc tcaggatgtc ggagcccctg cagagtgtgg tggaccacat ggccacccac 900
cttggggtgt ccccaagcag gatccttttg ctttttggag agacagagct atcacctact 960
gccactccca ggaccctaaa gctcggagtg gctgacatca ttgactgtgt ggtactaaca 1020
agttctccag aggccacaga gacgtcccaa cagctccagc tccgggtgca gggaaaggag 1080
aaacaccaga cactggaagt ctcactgtct cgagattccc ctctaaagac cctcatgtcc 1140
cactatgagg aggccatggg actgtcggga cggaagctct ccttcttctt tgatgggaca 1200
aagctttcag gcagggagct gccagctgac ctgggcatgg aatctgggga cctcattgag 1260
gtctggggct gacaccccac tccctgtttg acggcccagc ctggacttgg ggagaatgac 1320
tttccctttt ttgccccata agggctagca taagctgagg tagaacttat ctttaagctg 1380
cagcaaaatc aaggagtgac ttttgtcccc tctcctgttg accctggttt agagccgtta 1440
accacttggt gagttatgtg ggtgttgttg ccctgggtgg cctgtggctc cgtccacaag 1500
tcatgctgag ttttgcagcc tctgtgactt ggagatgtcc cttcacccct cccctttcac 1560
caccatcctc ttttcctcat ggaaatgtct gctttatgaa actatgcaca tattgaaagt 1620
gagttgaaac aaatgagggt tgggtaggag cttccaggcc tgggatttac accacgccta 1680
gcccagcaga ggccttagtc ccatttgggg cttgggagtg acatttgctt gaggcttata 1740
cactggtgtg gttgcctggc ttgcaggaaa tgaccaagct cacacatgct ggctgaagcg 1800
taagcagaca actgaggtac tcttttgaag gatgaaggtg gtggattctc agccctgggg 1860
gtcttcctca cctgaggacc cttcagagcc accctttcta gtttgcattt cctggtgcac 1920
acatttaagg cataacagca cattcatccc tttggtttgg gatctcagga atacagtccc 1980
atgcaaagat tctctggttt tatggctttt ttccctttct ttacaccatc ctctcccata 2040
agcacccatg tctttgaata tgaatgtatt tgtaaaatac cacgtttcat gtgtgaatat 2100
gtgcttttac tgtacatagt gctattgtgc aataggtctt atgctgtttt cactcaatgt 2160
gtgctaagat ctagccccat tgactcttct agaaatgcag tattgctttg acctgccatg 2220
tggcactcca caatgtcaat tgcagtttac acacattgcc taaagtgggg gacacctggg 2280
tgcccctgac cccttggcac cggatacagg ccacgataaa catcctttcg tgtgttccct 2340
tctgtgcttg tgtggcatgt gtacccagga tgggcctata ggtcacagag gtcagtttct 2400
ctttggtttt ccagattttc tttagaacgg tgactgaccc tcctacttga ggccgccctt 2460
ttctccttat ccttgccagc acttgtattg ccagactacc taatttttgc cagtctcatg 2520
ggtagatagt ggtgcagtgc tttaacatac attcatctga tcagcattaa tttggg     2576
```

<210> 7
<211> 1260
<212> DNA
<213> Homo sapiens

<220>
<221> gene
<222> (1)..(1260)
<223> NIP45-ORF

<400> 7

```
atggcggagc ctgtggggaa gcggggccgc tggtccggag gtagcggtgc cggccgaggg 60
ggtcggggcg gctggggcgg tcggggccgg gctcctcggg cccagcggtc tccatcccgg 120
ggcacgctgg acgtagtgtc tgtggacttg gtcaccgaca gcgatgagga aattctggag 180
gtcgccaccg ctcgcggtgc cgcggacgag gttgaggtgg agcccccgga gcccccgggg 240
ccggtcgcgt cccgggataa cagcaacagt gacagcgaag gggaggacag gcggcccgca 300
ggacccccgc gggagccggt caggcggcgg cggcggctgg tgctggatcc gggggaggcg 360
ccgctggttc cggtgtactc ggggaaggtt aaaagcagcc ttcgccttat cccagatgat 420
ctatccctcc tgaaactcta ccctccaggg gatgaggaag aggcagagct ggcagattcg 480
agtggtctct accatgaggg ctccccatca ccaggctctc cctggaagac aaagctgagg 540
actaaggata aagaagagaa gaaaaagaca gagtttctgg atctggacaa ctctcctctg 600
tccccacctt caccaaggac caaaagcaga acgcatactc gggcactcaa gaagttaagt 660
gaggtgaaca agcgcctcca ggatctccgt tcctgtctga gccccaagcc acctcagggt 720
caagagcaac agggccaaga ggatgaagtg gtcttggtgg aagggcccac cctcccagag 780
accccccgac tcttcccact caaaatccgt tgccgggctg acctggtcag attgcccctc 840
aggatgtcgg agcccctgca gagtgtggtg gaccacatgg ccacccacct tggggtgtcc 900
ccaagcagga tccttttgct ttttggagag acagagctat cacctactgc cactcccagg 960
accctaaagc tcggagtggc tgacatcatt gactgtgtgg tactaacaag ttctccagag 1020
gccacagaga cgtcccaaca gctccagctc cgggtgcagg gaaaggagaa acaccagaca 1080
ctggaagtct cactgtctcg agattcccct ctaaagaccc tcatgtccca ctatgaggag 1140
gccatgggac tgtcgggacg gaagctctcc ttcttctttg atgggacaaa gctttcaggc 1200
agggagctgc cagctgacct gggcatggaa tctggggacc tcattgaggt ctggggctga 1260
```

<210> 8
<211> 334
<212> PRT
<213> Homo sapiens

<220>
<221> PEPTIDE
<222> (1)..(334)
<223> NIP45V1

<400> 8

```
Met Ala Glu Pro Val Gly Lys Arg Gly Arg Trp Ser Gly Gly Ser Gly
  1               5                  10                  15

Ala Gly Arg Gly Gly Arg Gly Gly Trp Gly Gly Arg Gly Arg Ala Pro
             20                  25                  30

Arg Ala Gln Arg Ser Pro Ser Arg Gly Thr Leu Asp Val Val Ser Val
         35                  40                  45
```

```
Asp Leu Val Thr Asp Ser Asp Glu Glu Ile Leu Glu Val Ala Thr Ala
     50                  55                  60

Arg Gly Ala Ala Asp Glu Val Glu Val Glu Pro Pro Glu Pro Pro Gly
 65                  70                  75                  80

Pro Val Ala Ser Arg Asp Asn Ser Asn Ser Asp Ser Glu Gly Glu Asp
                 85                  90                  95

Arg Arg Pro Ala Gly Pro Pro Arg Glu Pro Val Arg Arg Arg Arg Arg
            100                 105                 110

Leu Val Leu Asp Pro Gly Glu Ala Pro Leu Val Pro Val Tyr Ser Gly
        115                 120                 125

Lys Val Lys Ser Ser Leu Arg Leu Ile Pro Asp Asp Leu Ser Leu Leu
    130                 135                 140

Lys Leu Tyr Pro Pro Gly Asp Glu Glu Glu Ala Glu Leu Ala Asp Ser
145                 150                 155                 160

Ser Gly Leu Tyr His Glu Gly Ser Pro Ser Pro Gly Ser Pro Trp Lys
                165                 170                 175

Thr Lys Leu Arg Thr Lys Asp Lys Glu Glu Lys Lys Lys Thr Glu Phe
            180                 185                 190

Leu Asp Leu Asp Asn Ser Pro Leu Ser Pro Pro Ser Pro Arg Thr Lys
        195                 200                 205

Ser Arg Thr His Thr Arg Ala Leu Lys Lys Leu Ser Glu Val Asn Lys
    210                 215                 220

Arg Leu Gln Asp Leu Arg Ser Cys Leu Ser Pro Lys Pro Pro Gln Gly
225                 230                 235                 240

Gln Glu Gln Gln Gly Gln Glu Asp Glu Val Val Leu Val Glu Gly Pro
                245                 250                 255

Thr Leu Pro Glu Thr Pro Arg Leu Phe Pro Leu Lys Ile Arg Cys Arg
            260                 265                 270

Ala Asp Leu Val Arg Leu Pro Leu Arg Met Asp Ser Pro Leu Lys Thr
        275                 280                 285

Leu Met Ser His Tyr Glu Glu Ala Met Gly Leu Ser Gly Arg Lys Leu
    290                 295                 300
```

Ser Phe Phe Phe Asp Gly Thr Lys Leu Ser Gly Arg Glu Leu Pro Ala
305 310 315 320

Asp Leu Gly Met Glu Ser Gly Asp Leu Ile Glu Val Trp Gly
325 330

<210> 9
<211> 286
<212> PRT
<213> Homo sapiens

<220>
<221> PEPTIDE
<222> (1)..(286)
<223> NIP45V2

<400> 9

```
Met Ala Glu Pro Val Gly Lys Arg Gly Arg Trp Ser Gly Gly Ser Gly
 1               5                  10                  15

Ala Gly Arg Gly Gly Arg Gly Gly Trp Gly Gly Arg Gly Arg Ala Pro
            20                  25                  30

Arg Ala Gln Arg Ser Pro Ser Arg Gly Thr Leu Asp Val Val Ser Val
        35                  40                  45

Asp Leu Val Thr Asp Ser Asp Glu Glu Ile Leu Glu Val Ala Thr Ala
    50                  55                  60

Arg Gly Ala Ala Asp Glu Val Glu Val Glu Pro Pro Glu Pro Pro Gly
65                  70                  75                  80

Pro Val Ala Ser Arg Asp Asn Ser Asn Ser Asp Ser Glu Gly Glu Asp
                85                  90                  95

Arg Arg Pro Ala Gly Pro Pro Arg Glu Pro Val Arg Arg Arg Arg Arg
            100                 105                 110

Leu Val Leu Asp Pro Gly Glu Ala Pro Leu Val Pro Val Tyr Ser Gly
        115                 120                 125

Lys Val Lys Ser Ser Leu Arg Leu Ile Pro Asp Asp Leu Ser Leu Leu
    130                 135                 140

Lys Leu Tyr Pro Pro Gly Asp Glu Glu Glu Ala Glu Leu Ala Asp Ser
145                 150                 155                 160
```

Ser Gly Leu Tyr His Glu Gly Ser Pro Ser Pro Gly Ser Pro Trp Lys
165 170 175

Thr Lys Leu Arg Thr Lys Asp Lys Glu Glu Lys Lys Lys Thr Glu Phe
180 185 190

Leu Asp Leu Asp Asn Ser Pro Leu Ser Pro Pro Ser Pro Arg Thr Lys
195 200 205

Ser Arg Thr His Thr Arg Ala Leu Lys Lys Leu Ser Glu Val Asn Lys
210 215 220

Arg Leu Gln Asp Leu Arg Ser Cys Leu Ser Pro Lys Pro Pro Gln Gly
225 230 235 240

Gln Glu Gln Gln Gly Gln Glu Asp Glu Val Val Leu Val Glu Gly Pro
245 250 255

Thr Leu Pro Glu Thr Pro Arg Leu Phe Pro Leu Lys Ile Arg Cys Arg
260 265 270

Ala Asp Leu Val Arg Leu Pro Leu Arg Met Thr Val Trp Tyr
275 280 285

<210> 10
<211> 303
<212> PRT
<213> Homo sapiens

<220>
<221> PEPTIDE
<222> (1)..(303)
<223> NIP45V3

<400> 10

```
Trp Arg Ser Leu Trp Gly Ser Gly Ala Ala Gly Pro Glu Val Lys Ser
 1               5                  10                  15

Ser Leu Arg Leu Ile Pro Asp Asp Leu Ser Leu Leu Lys Leu Tyr Pro
            20                  25                  30

Pro Gly Asp Glu Glu Glu Ala Glu Leu Ala Asp Ser Ser Gly Leu Tyr
        35                  40                  45

His Glu Gly Ser Pro Ser Pro Gly Ser Pro Trp Lys Thr Lys Leu Arg
    50                  55                  60
```

```
Thr Lys Asp Lys Glu Glu Lys Lys Lys Thr Glu Phe Leu Asp Leu Asp
 65                  70                  75                  80

Asn Ser Pro Leu Ser Pro Pro Ser Pro Arg Thr Lys Ser Arg Thr His
                 85                  90                  95

Thr Arg Ala Leu Lys Lys Leu Ser Glu Val Asn Lys Arg Leu Gln Asp
            100                 105                 110

Leu Arg Ser Cys Leu Ser Pro Lys Pro Pro Gln Gly Gln Glu Gln Gln
        115                 120                 125

Gly Gln Glu Asp Glu Val Val Leu Val Glu Gly Pro Thr Leu Pro Glu
    130                 135                 140

Thr Pro Arg Leu Phe Pro Leu Lys Ile Arg Cys Arg Ala Asp Leu Val
145                 150                 155                 160

Arg Leu Pro Leu Arg Met Ser Glu Pro Leu Gln Ser Val Val Asp His
                165                 170                 175

Met Ala Thr His Leu Gly Val Ser Pro Ser Arg Ile Leu Leu Leu Phe
            180                 185                 190

Gly Glu Thr Glu Leu Ser Pro Thr Ala Thr Pro Arg Thr Leu Lys Leu
        195                 200                 205

Gly Val Ala Asp Ile Ile Asp Cys Val Val Leu Thr Ser Ser Pro Glu
    210                 215                 220

Ala Thr Glu Thr Ser Gln Gln Leu Gln Leu Arg Val Gln Gly Lys Glu
225                 230                 235                 240

Lys His Gln Thr Leu Glu Val Ser Leu Ser Arg Asp Ser Pro Leu Lys
                245                 250                 255

Thr Leu Met Ser His Tyr Glu Glu Ala Met Gly Leu Ser Gly Arg Lys
            260                 265                 270

Leu Ser Phe Phe Phe Asp Gly Thr Lys Leu Ser Gly Arg Glu Leu Pro
        275                 280                 285

Ala Asp Leu Gly Met Glu Ser Gly Asp Leu Ile Glu Val Trp Gly
    290                 295                 300
```

<210> 11
<211> 55
<212> PRT
<213> Homo sapiens

<220>
<221> PEPTIDE
<222> (1)..(55)
<223> NIP95V4

<400> 11

```
Met Arg Lys Asp Ser Pro Leu Lys Thr Leu Met Ser His Tyr Glu Glu
 1               5                  10                  15

Ala Met Gly Leu Ser Gly Arg Lys Leu Ser Phe Phe Phe Asp Gly Thr
            20                  25                  30

Lys Leu Ser Gly Arg Glu Leu Pro Ala Asp Leu Gly Met Glu Ser Gly
        35                  40                  45

Asp Leu Ile Glu Val Trp Gly
    50                  55
```

<210> 12
<211> 419
<212> PRT
<213> Homo sapiens

<220>
<221> PEPTIDE
<222> (1)..(419)
<223> NIP45

<400> 12

```
Met Ala Glu Pro Val Gly Lys Arg Gly Arg Trp Ser Gly Gly Ser Gly
  1               5                  10                  15

Ala Gly Arg Gly Gly Arg Gly Gly Trp Gly Gly Arg Gly Arg Ala Pro
             20                  25                  30

Arg Ala Gln Arg Ser Pro Ser Arg Gly Thr Leu Asp Val Val Ser Val
         35                  40                  45

Asp Leu Val Thr Asp Ser Asp Glu Glu Ile Leu Glu Val Ala Thr Ala
     50                  55                  60

Arg Gly Ala Ala Asp Glu Val Glu Val Glu Pro Pro Glu Pro Pro Gly
 65                  70                  75                  80
```

Pro Val Ala Ser Arg Asp Asn Ser Asn Ser Asp Ser Glu Gly Glu Asp
85 90 95

Arg Arg Pro Ala Gly Pro Pro Arg Glu Pro Val Arg Arg Arg Arg Arg
100 105 110

Leu Val Leu Asp Pro Gly Glu Ala Pro Leu Val Pro Val Tyr Ser Gly
115 120 125

Lys Val Lys Ser Ser Leu Arg Leu Ile Pro Asp Asp Leu Ser Leu Leu
130 135 140

Lys Leu Tyr Pro Pro Gly Asp Glu Glu Glu Ala Glu Leu Ala Asp Ser
145 150 155 160

Ser Gly Leu Tyr His Glu Gly Ser Pro Ser Pro Gly Ser Pro Trp Lys
165 170 175

Thr Lys Leu Arg Thr Lys Asp Lys Glu Glu Lys Lys Lys Thr Glu Phe
180 185 190

Leu Asp Leu Asp Asn Ser Pro Leu Ser Pro Pro Ser Pro Arg Thr Lys
195 200 205

Ser Arg Thr His Thr Arg Ala Leu Lys Lys Leu Ser Glu Val Asn Lys
210 215 220

Arg Leu Gln Asp Leu Arg Ser Cys Leu Ser Pro Lys Pro Pro Gln Gly
225 230 235 240

Gln Glu Gln Gln Gly Gln Glu Asp Glu Val Val Leu Val Glu Gly Pro
245 250 255

Thr Leu Pro Glu Thr Pro Arg Leu Phe Pro Leu Lys Ile Arg Cys Arg
260 265 270

Ala Asp Leu Val Arg Leu Pro Leu Arg Met Ser Glu Pro Leu Gln Ser
275 280 285

Val Val Asp His Met Ala Thr His Leu Gly Val Ser Pro Ser Arg Ile
290 295 300

Leu Leu Leu Phe Gly Glu Thr Glu Leu Ser Pro Thr Ala Thr Pro Arg
305 310 315 320

Thr Leu Lys Leu Gly Val Ala Asp Ile Ile Asp Cys Val Val Leu Thr
325 330 335

Ser Ser Pro Glu Ala Thr Glu Thr Ser Gln Gln Leu Gln Leu Arg Val
340 345 350

Gln Gly Lys Glu Lys His Gln Thr Leu Glu Val Ser Leu Ser Arg Asp
355 360 365

Ser Pro Leu Lys Thr Leu Met Ser His Tyr Glu Glu Ala Met Gly Leu
370 375 380

Ser Gly Arg Lys Leu Ser Phe Phe Phe Asp Gly Thr Lys Leu Ser Gly
385 390 395 400

Arg Glu Leu Pro Ala Asp Leu Gly Met Glu Ser Gly Asp Leu Ile Glu
405 410 415

Val Trp Gly

<210> 13
<211> 24
<212> DNA
<213> Artificial Sequence

<220>
<223> Description of Artificial Sequence: Primer

<400> 13
cccgactctt cccactcaaa atcc 24

<210> 14
<211> 24
<212> DNA
<213> Artificial Sequence

<220>
<223> Description of Artificial Sequence: Primer

<400> 14
cagtcccatg gcctcctcat agtg 24

<210> 15
<211> 24
<212> DNA
<213> Artificial Sequence

<220>
<223> Description of Artificial Sequence: Primer

<400> 15
aaagtgtgcc atggcggagc ctgt 24

<210> 16

<211> 23
<212> DNA
<213> Artificial Sequence

<220>
<223> Description of Artificial Sequence: Primer

<400> 16
gggtgtcagc cccagacctc aat 23

## Description

### TECHNICAL FIELD OF THE INVENTION

The present invention relates to nucleic acid and amino acid sequences of a novel NF-AT interacting protein NIP 45 variant and its use in diagnosis and therapy for human disease.

### BACKGROUND OF THE INVENTION

Many immunologically-mediated clinical diseases including autoimmune diseases, allergic diseases, and infectious diseases are reported to be highly relevant to the ratio of CD4+ T helper cell type 1 (Th1) to CD4+ T helper cell type 2 (Th2) (Heinzel, F.P., et al., (1989) J. Exp. Med. 169: 59-72; Pearce, E.J., et al. (1991) J. Exp. Med. 173: 159-166; Shearer, G.M. and Clerici, M. (1992) Prog. Chem. Immunol. 54: 21-43). To alter or regulate the ratios of Th1 and Th 2 cells, therefore, may give a clue to treat or prevent immunologically-mediated diseases.

The mechanisms by which the Th1 and Th 2 ratio is determined involve the differentiation of CD4 T helper precursor cells (Thp) to choose to become Th1 or Th 2 effector cells. The differentiation is partly regulated by cytokines, such as IL-2, IL-4, and IL-12, whose expression can be induced by transcription factors, some of the most important of which are proteins of the NFAT (Nuclear Factor of Activated T cells) family. NFAT proteins are expressed in most immune cell types and play an important role in the transcription of many cytokines, such as IL-2, IL-3, IL-4, IL-5, IL-13, GM-CSF, IFN-$\gamma$, and TNF-$\alpha$, as well as several other genes involved in immune cell responses.To increase or decrease the level of selected cytokines is one way to regulate Th1 to Th2 ratio.

Recently, a protein of 45 kDa derived from murine tissue that interacts with members of the NFAT family of proteins has been isolated and termed NIP45 (Hodge MR, Chun HJ, Rengarajan J, Alt A, Lieberson R, Glimcher LH. NF-AT-Driven interleukin-4 transcription potentiated by NIP45. Science, 1996 Dec 13; 274(5294): 1903-5; Hodge MR et al., WO 97/39721, USP 5,858,711, and USP 5,958,671). Further, WO 99/21993 discloses human NIP45 polypeptide and polynucleotide sequences.

NIP45 has been shown to interact with NFATp and to potentiate the transcription of the IL-4 gene induced by the binding of NFATp to the IL-4 gene promoter. The nature of this interaction is unclear, but one possibility is that NIP45 is an accessory protein that is involved in the transport of NFATp from the cytoplasm into the nucleus where it can bind to the IL-4 gene promoter. In the absence of such an accessory protein, NFATp may be inactive because of its inability to cross through the nuclear membrane on its own. Experiments with NFATp knockout mice have shown that NFATp deficiency leads to the accumulation of peripheral T cells with a preactivated phenotype, enhanced immune responses of T cells after secondary stimulation in vitro, and severe defects in the proper termination of antigen responses (Schuh, K. et al. (1998) Eur. J. Immunol. 28(8): 2456-66). In some of these mice, large germinal centers develop in the spleen and peripheral lymph nodes and there is a pronounced retardation in the involution of the thymus.

There is a need in the art to identify additional members of the NFAT interacting protein variant whose activity can be regulated to provide therapeutic effects, particularly for diseases and conditions involving immunologically-mediated responses.

### SUMMARY OF THE INVENTION

It is an object of the invention to provide reagents and methods of regulating NF-AT interacting proteins. This and others objects of the invention are provided by one or more of the embodiments described below.

One embodiment of the invention is a NIP45V1 polypeptide comprising:

> the amino acid sequence shown in SEQ ID NO: 8 lacking amino acid residues 283 through 367 with respect to the fall length NIP45 protein.

Yet another embodiment of the invention is a method of screening for agents which decrease the activity of NIP45V1. A test compound is contacted with a NIP45V1 polypeptide comprising:

> the amino acid sequence shown in SEQ ID NO: 8 lacking amino acid residues 283 through 367 with respect to the fall length NIP45 protein.

Binding between the test compound and the NIP45V1 polypeptide is detected. A test compound which binds to the NIP45V1 polypeptide is thereby identified as a potential agent for decreasing the activity of NIP45V1.

Another embodiment of the invention is a method of screening for agents which decrease the activity of NIP45V1. A test compound is contacted with a polynucleotide encoding a NIP45V1 polypeptide, wherein the polynucleotide comprises a nucleotide sequence

the nucleotide sequence shown in SEQ ID NO. 2 lacking the sequence part coding for amino acid residues 283 through 367.

Binding of the test compound to the polynucleotide is detected. A test compound which binds to the polynucleotide is identified as a potential agent for decreasing the activity of NIP45V1. The agent can work by decreasing the amount of the NIP45V1 through interacting with the NIP45V1 mRNA.

Another embodiment of the invention is a method of screening for agents which regulate the activity of NIP45V1. A test compound is contacted with a NIP45V1 polypeptide comprising:

the amino acid sequence shown in SEQ ID NO: 8 lacking amino acid residues 283 through 367 with respect to the fall length NIP45 protein,

A NIP45V1 activity of the polypeptide is detected. A test compound which increases NIP45V1 activity of the polypeptide relative to NIP45V1 activity in the absence of the test compound is thereby identified as a potential agent for increasing the activity of NIP45V1 A test compound which decreases NIP45V activity of the polypeptide relative to NIP45V1 activity in the absence of the test compound is thereby identified as a potential agent for decreasing the activity of NIP45V1.

Even another embodiment of the invention is a method of screening for agents which decrease the activity of NIP45V1. A test compound is contacted with a NIP45V1 product of a polynucleotide which comprises:

the nucleotide sequence shown in SEQ ID NO. 2 lacking the sequence part coding for amino acid residues 283 through 367.

Binding of the test compound to the NIP45V1 product is detected. A test compound which binds to the NIP45V1 product is thereby identified as a potential agent for decreasing the activity of NIP45V1.

Still another embodiment of the invention is a method of reducing the activity of NIP45V1. A cell is contacted with a reagent which specifically binds to a polynucleotide encoding a NIP45V1 polypeptide or the product encoded by the polynucleotide, wherein the polynucleotide comprises:

the nucleotide sequence shown in SEQ ID NO. 2 lacking the sequence part coding for amino acid residues 283 through 367.

NIP45V1 activity in the cell is thereby decreased.

## BRIEF DESCRIPTION OF THE DRAWING

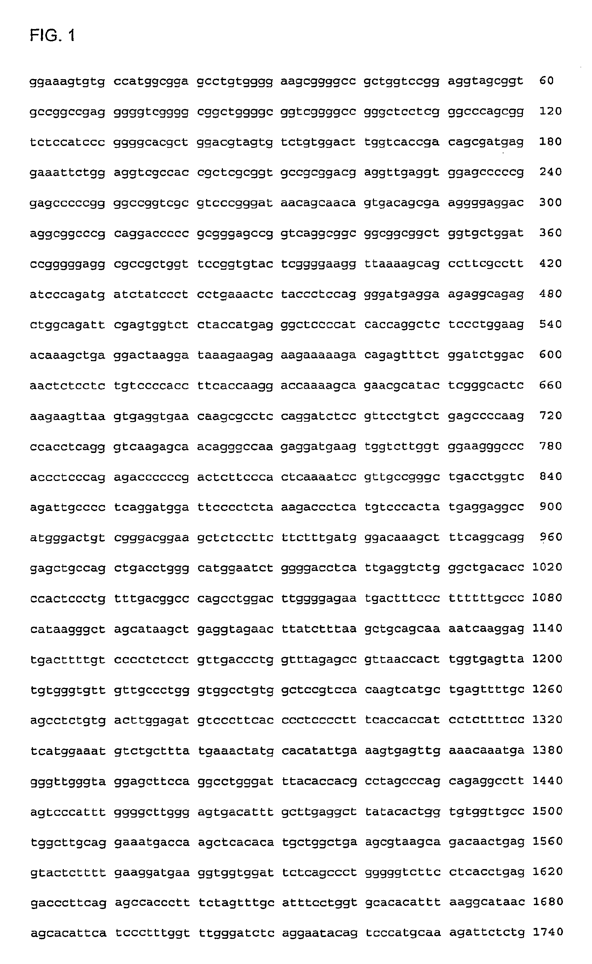

Fig. 1 shows the DNA-sequence encoding a NIP45V1 polypeptide.

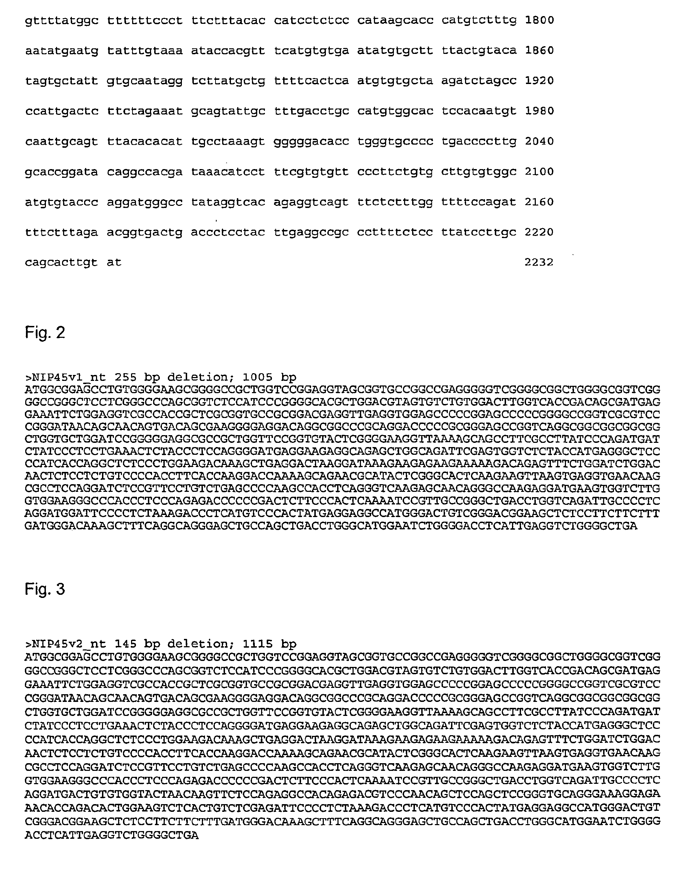

Fig. 2 shows the DNA-sequence (ORF) encoding a NIP45V1 polypeptide.
Fig. 3 shows the DNA-sequence encoding a NIP45V2 polypeptide.

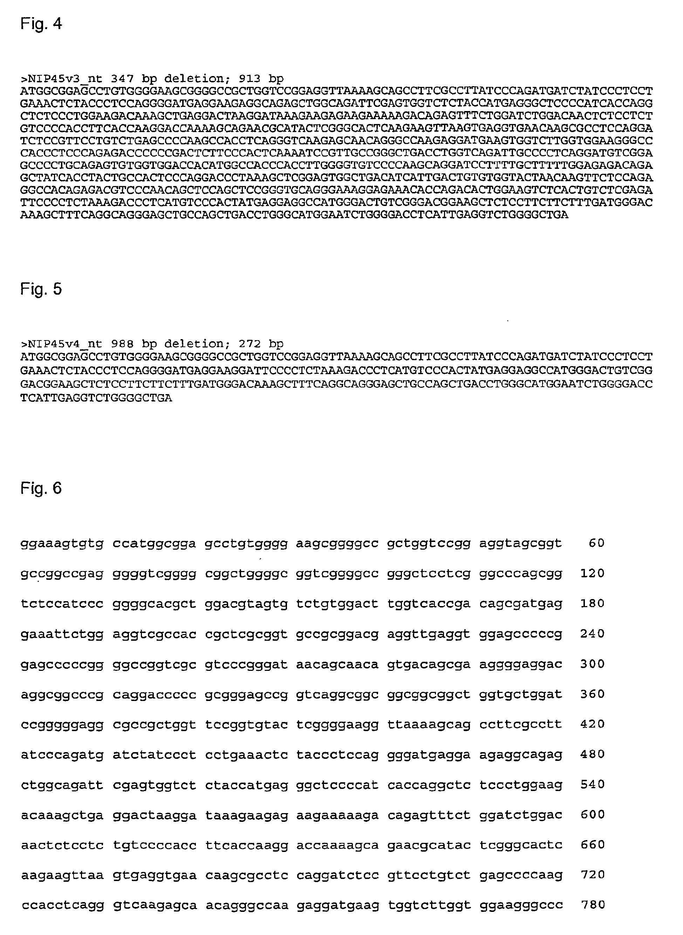

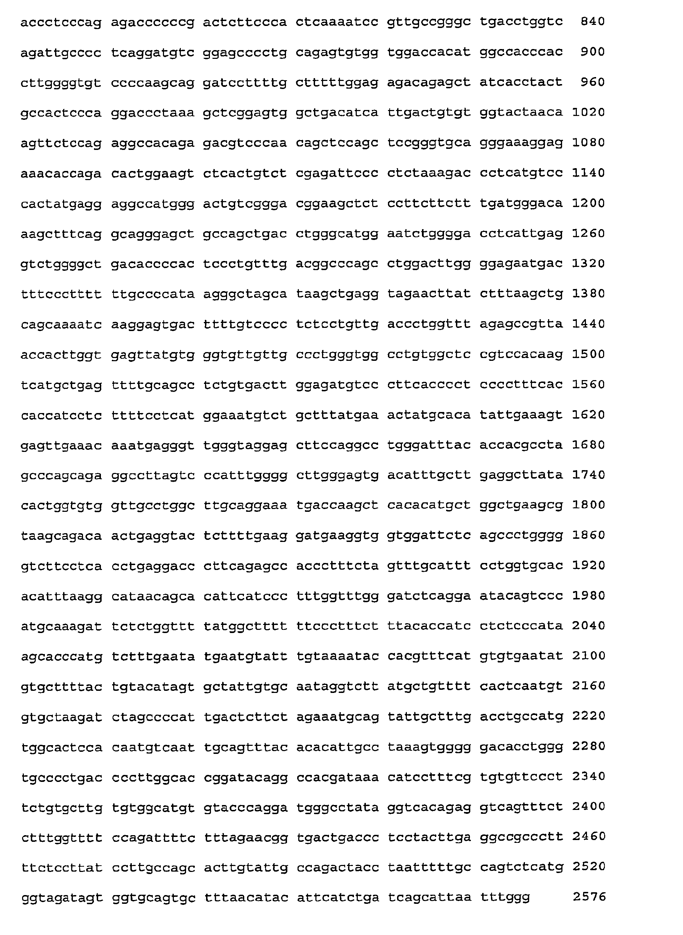

Fig. 4 shows the DNA-sequence encoding a NIP45V3 polypeptide.
Fig. 5 shows the DNA-sequence encoding a NIP45V4 polypeptide.
Fig. 6 shows the DNA-sequence encoding NIP45.

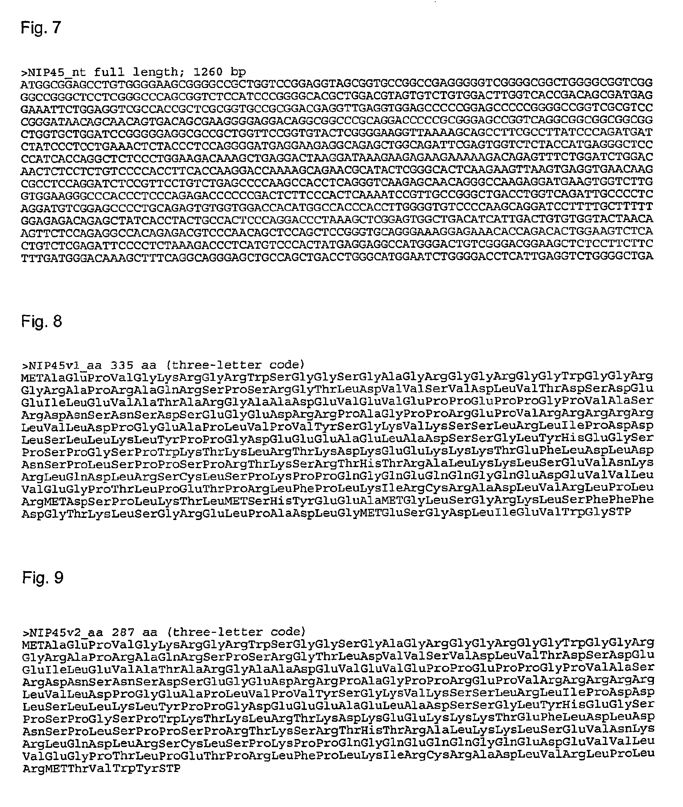

Fig. 7 shows the DNA-sequence (ORF) encoding NIP45.
Fig. 8 shows the amino acid sequence deduced from the DNA-sequence of Fig.2.
Fig. 9 shows the amino acid sequence deduced from the DNA-sequence of Fig.3.

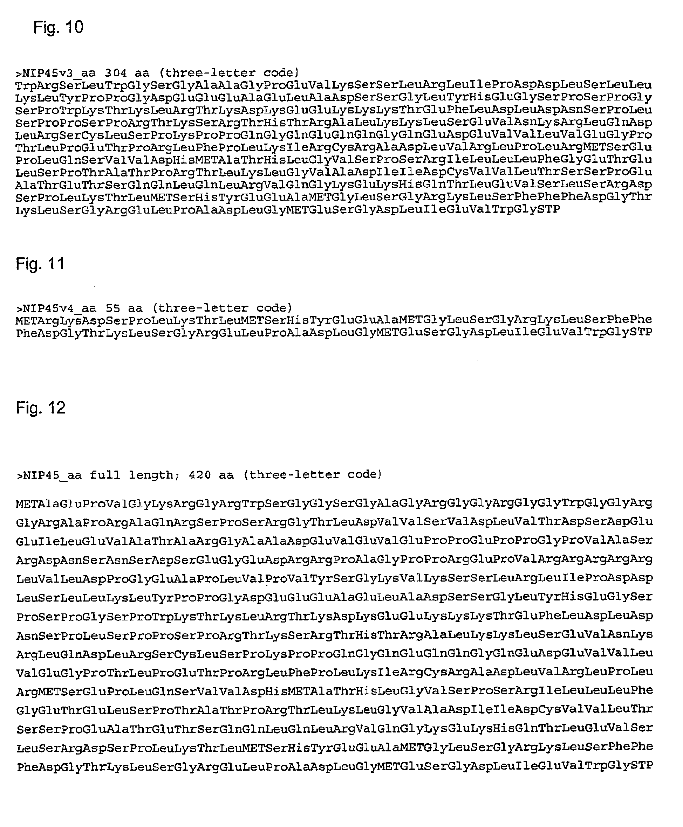

Fig. 10 shows the amino acid sequence deduced from the DNA-sequence of Fig.4.
Fig. 11 shows the amino acid sequence deduced from the DNA-sequence of Fig.5.
Fig. 12 shows the amino acid sequence deduced from the DNA-sequence of Fig. 6.
Fig. 13 shows PCR amplified bands of NIP45 and NIP45V in various immune related tissues.

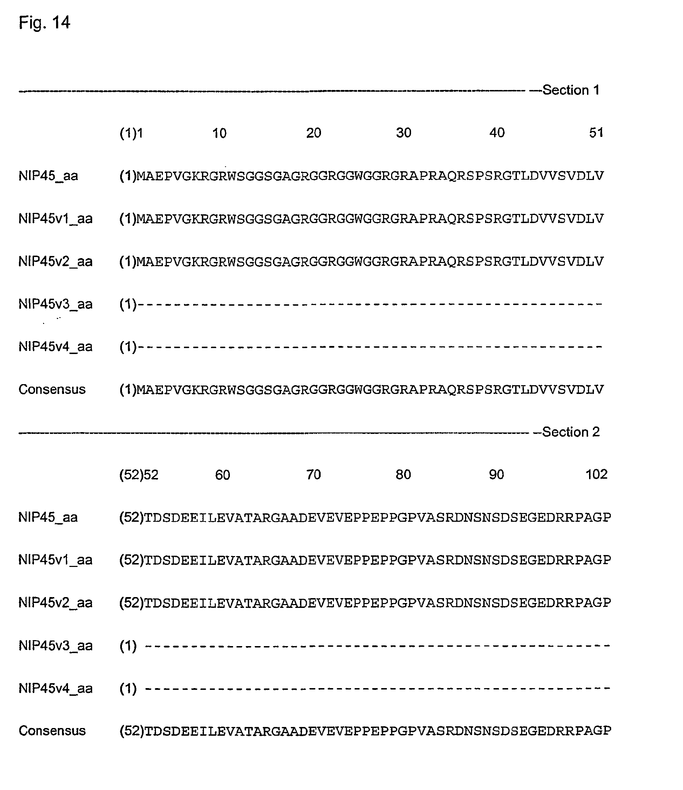

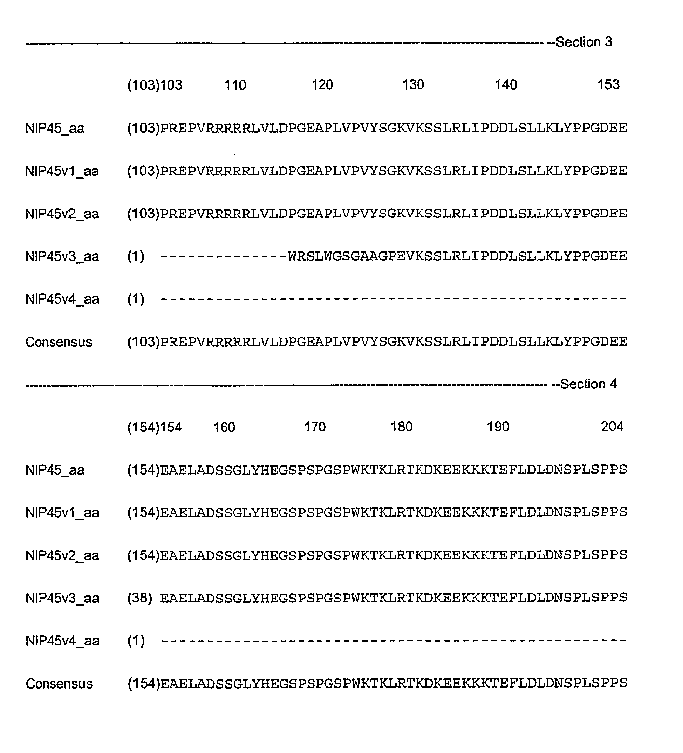

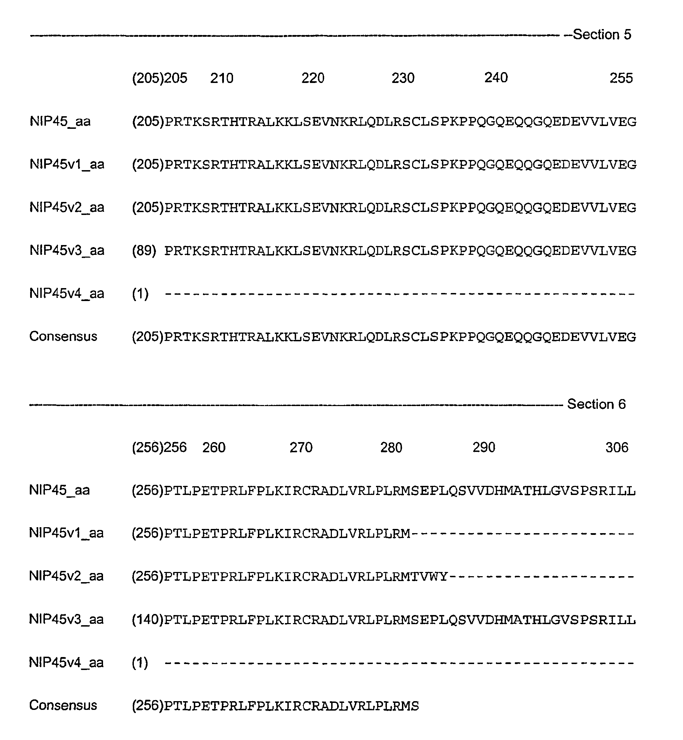

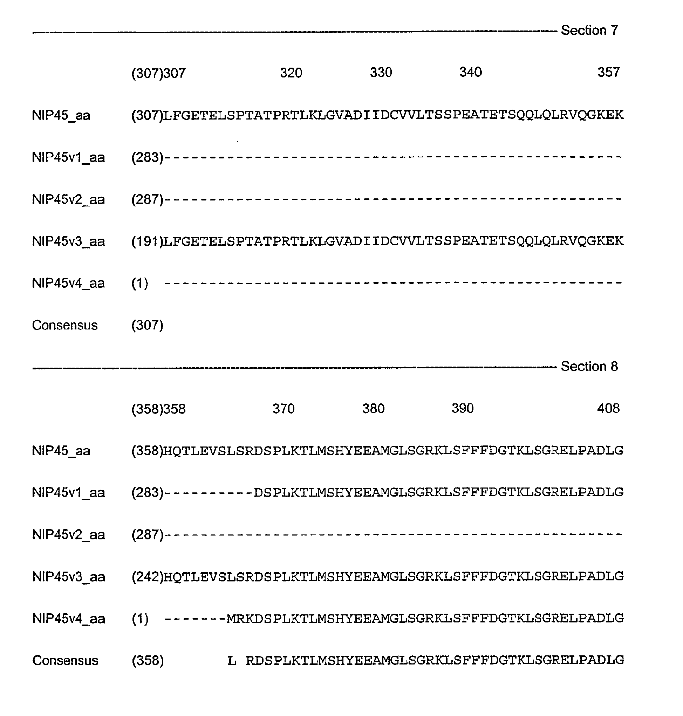

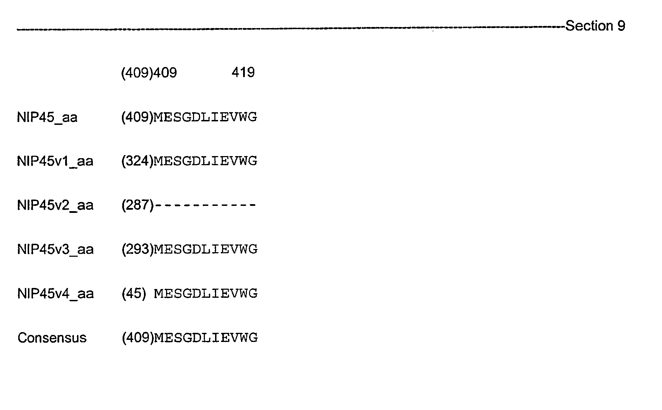

Fig. 14 shows the alignment of NIP 45 V (v1, 2, 3, and 4 proteins) and NIP 45.

## DETAILED DESCRIPTION OF THE INVENTION

The invention relates to an isolated polynucleotide encoding a NIP45V1 polypeptide comprising:

a) the amino acid sequence shown in SEQ ID NO: 8 lacking amino acid residues 283 through 367 with respect to the fall length NIP45 protein.

b) a polynucleotide comprising the sequence of SEQ ID NO.2 lacking the sequence part coding for amino acid residues 283 through 367.

Furthermore, it has been discovered by the present applicant that NIP 45 splice variant, particularly human NIP45 splice variant, activity can be regulated to control autoimmune diseases, allergic diseases, and infectious diseases, and other chronic inflammatory diseases. Such diseases include asthma, allergic rhinitis, atopic dermatitis, hives, conjunctivitis, vernal catarrh, chronic arthrorheumatism, systemic lupus erythematosus, myasthenia gravis, psoriasis, diabrotic colitis, systemic inflammatory response syndrome (SIRS), llymphofollicular thymitis, sepsis, polymyositis, dermatomyositis, polyaritis nodoa, mixed connective tissue disease (MCTD), Sjoegren's syndrome, gout, and the like.

NIP45 is known to interact with NFATp and to potentiate the transcription of the IL-4 gene induced by the binding of NFATp to the IL-4 gene promoter. The nature of this interaction is unclear, but one possibility is that NIP45 is an accessory protein that is involved in the transport of NFATp from the cytoplasm into the nucleus where it can bind to the IL-4 gene promoter. In the absence of such an accessory protein, NFATp may be inactive because of its inability to cross through the nuclear membrane on its own. Experiments with NFATp knockout mice have shown that NFATp deficiency leads to the accumulation of peripheral T cells with a preactivated phenotype, enhanced immune responses of T cells after secondary stimulation in vitro, and severe defects in the proper termination of antigen responses. In some of these mice, large germinal centers develop in the spleen and peripheral lymph nodes and there is a pronounced retardation in the involution of the thymus.

The expression of a NIP45vl splice variant of NIP45 in the thymus, spleen, and lymph nodes may play an important role in the normal formation of germinal centers in these lymphoid organs. The NIP45vl splice variant lacks a large portion of the full-length NIP45 protein, most significant of which are regions of homology to Ubiquitin and Sentrin molecules. Ubiquitins are involved in the regulated turnover of proteins required for controlling cell cycle progression. Sentrins are small ubiquitin-like proteins that are thought to be covalently attached to other proteins to mark them for transport into the nucleus. Lack of the Ubiqitin-homology domain and part of the Sentrin-homolgy domain may make NIP45v1 ineffective at transporting NFATp into the nucleus (Kamitani, T. et al. (1997) J. Biol. Chem. 272 (22): 14001-4; Okura, T. et al. (1996) J. Immunol. 157 (10): 4277-81). Similarly, the deletion in NIP45v1 may alter the interaction with NFATp so that the coexpression of NIP45v1 and NFATp no longer has a synergistic effect on transcription via the IL-4 promoter. The effect, therefore, of NIP45v1 in the cell may be to disable NFATp or to block the interaction between NIP45 and NFATp and thereby promote the formation of germinal centers, preactivate T cells, enhance secondary immune responses, and delay termination of antigen responses.

On the other hand, the deletion in NIP45v1 may bring together two parts of the NIP45 molecule to enhance the function of NIP45v1 in such a way that its interaction with NFATp has a greater enhancing effect on IL-4 transcription than that of the full-length NIP45.

The increased formation of germinal centers in the thymus is a property of human patients with lymphofollicular thymitis and is often connected with the development of autoimmune diseases such as myasthenia gravis (Muller-Hermelink, H.K., Marx, A. Kirchner, T. Thymus and mediastinum. In Damjanov, I, Linder, J (eds.), Mosby-Year Book. Mosby, St. Louis 1996, pp.1218-43). Additionally, NFATp deficient mice exhibit a strong tendency toward the development of Th2 type immune responses, with paradoxically strong enhancement of the transcription of several Th2 type genes, including IL-4, IL-5, and IL-13 (Kiani, A. et al.(1997) Immunity 7(6): 849-60). This preferential development of Th2 type immune responses may lead to overactive allergic responses and Th2-dependent autoimmune diseases and other disorders.

The NIP45 alternative splice variant 1 can be used as a target to develop selective inhibitors or activators directed against the variant.

*NIP45 VPolypeptides*

NIP45V1 polypeptides according to the present invention comprise the amino acid sequence shown in SEQ ID NO.8 (NIP 45 v1) lacking amino acid residues 283 through 367 with respect to the fall length NIP45 protein.

*Biologically Active Variants*

Percent identity between a putative NIP45 V variant and an amino acid sequence of SEQ ID NO.8 is determined using the Blast2 alignment program.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of an NIP 45 V1 polypeptide can be found using computer programs well known in the art, such as DNASTAR software. Whether an amino acid change results in a biologically active NIP45 variant polypeptide can readily be determined by assaying for NIP 45 V1 polypeptide activity, as described, for example, in the specific examples, below.

*Fusion Proteins*

Fusion proteins can comprise at least 5, 6, 8, 10, 25, or 50 or more contiguous amino acids of an amino acid sequence shown in any of SEQ ID NO.8 Fusion proteins are useful for generating antibodies against NIP45 V1 polypeptide amino acid sequences and for use in various assay systems. For example, fusion proteins can be used to identify proteins which interact with portions of an NIP 45 V1 polypeptide. Protein affinity chromatography or library-based assays for protein-protein interactions, such as the yeast two-hybrid or phage display systems, can be used for this purpose. Such methods are well known in the art and also can be used as drug screens.

An NIP 45 V1 polypeptide fusion protein comprises two polypeptide segments fused together by means of a peptide bond. The first polypeptide segment comprises at least 5, 6, 8, 10, 25, or 50 or more contiguous amino acids of any of SEQ ID NO.8 or of a biologically active variant, such as those described above. The first polypeptide segment also can comprise full-length NIP45-variant.

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the NIP 45 V1 polypeptide-encoding sequence and the heterologous protein sequence, so that the NIP 45 V1 polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from the group consisting of SEQ ID NO.2 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

*Identification of Species Homologs*

Species homologs of the NIP 45 V1 polypeptide can be obtained using NIP 45 V1 polypeptide polynucleotides (described below) to make suitable probes or primers for screening cDNA expression libraries from other species, such as mice, monkeys, or yeast, identifying cDNAs which encode homologs of the NIP 45 V1 polypeptide, and expressing the cDNAs as is known in the art.

*NIP 45 V Polynucleotides*

An NIP45 like polynucleotide can be single- or double-stranded, and comprise a coding sequence or the complement of a coding sequence for an NIP 45 V1 polypeptide. The coding sequence for human NIP45 V1 polypeptide is shown in SEQ ID NO.2.

Degenerate nucleotide sequences encoding human NIP 45 V1 polypeptides, as well as homologous nucleotide sequences which are at least about 50, preferably about 75, 85, 90, 95, 96, 98 or 99% identical to the nucleotide sequence shown in SEQ ID NO. 2 also are NIP 45 V polynucleotides. Percent sequence identity between the sequences of two polynucleotides is determined using computer programs such as ALIGN which employ the PASTA algorithm, using an affine gap search with a gap open penalty of -12 and a gap extension penalty of -2. Complementary DNA (cDNA) molecules, species homologs, and variants of NIP 45 V1 polynucleotides which encode biologically active NIP45 V1 polypeptides also are NIP 45 V1 polynucleotides.

*Identification of Variants and Homologs of NIP 45 V Polynucleotides*

Variants and homologs of the NIP 45 V1 polynucleotides described above also are NIP 45 V1 polynucleotides. Typically, homologous NIP 45 V1 polynucleotide sequences can be identified by hybridization of candidate polynucleotides to known NIP 45 V1 polynucleotides under stringent conditions, as is known in the art. For example, using the following wash conditions--2X SSC (0.3 M NaCl, 0.03 M sodium citrate, pH 7.0), 0.1% SDS, room temperature twice, 30 minutes each; then 2X SSC, 0.1% SDS, 50°C once, 30 minutes; then 2X SSC, room temperature twice, 10 minutes each--homologous sequences can be identified which contain at most about 25-30% basepair mismatches. More preferably, homologous nucleic acid strands contain 15-25% basepair mismatches, even more preferably 5-15% basepair mismatches.

Species homologs of the NIP 45 V1 polynucleotides disclosed herein also can be identified by making suitable probes or primers and screening cDNA expression libraries from other species, such as mice, monkeys, or yeast. Human variants of NIP 45 V1 polynucleotides can be identified, for example, by screening human cDNA expression libraries. It is well known that the $T_m$ of a double-stranded DNA decreases by 1-1.5 °C with every 1% decrease in homology (Bonner *et al., J. Mol. Biol. 81,* 123 (1973). Variants of human NIP 45 V1 polynucleotides or NIP45 V1 polynucleotides of other species can therefore be identified by hybridizing a putative homologous NIP 45 V1 polynucleotide with a polynucleotide having a nucleotide sequence of any of SEQ ID NO.2 or the complement thereof to form a test hybrid. The melting temperature of the test hybrid is compared with the melting temperature of a hybrid comprising transformylase polynucleotides having perfectly complementary nucleotide sequences, and the number or percent of basepair mismatches within the test hybrid is calculated.

Nucleotide sequences which hybridize to transformylase polynucleotides or their complements following stringent hybridization and/or wash conditions also are NIP 45 V1 polynucleotides. Stringent wash conditions are well known and understood in the art and are disclosed, for example, in Sambrook *et al.*, MOLECULAR CLONING: A LABORATORY MANUAL, 2d ed., 1989, at pages 9.50-9.51.

Typically, for stringent hybridization conditions a combination of temperature and salt concentration should be chosen that is approximately 12-20°C below the calculated $T_m$ of the hybrid under study. The $T_m$ of a hybrid between an NIP 45 V1 polynucleotide having a nucleotide sequence shown in any of SEQ ID NO.2 or the complement thereof and a polynucleotide sequence which is at least about 50, preferably about 75, 90, 96, or 98% identical to one of those nucleotide sequences can be calculated, for example, using the equation of Bolton and McCarthy, *Proc. Natl. Acad Sci. U.S.A.* 48, 1390 (1962):

$$T_m = 81.5\ °C - 16.6(\log_{10}[Na^+]) + 0.41(\%G + C) - 0.63(\%formamide) - 600/l,$$

where *l* = the length of the hybrid in basepairs.

Stringent wash conditions include, for example, 4X SSC at 65°C, or 50% formamide, 4X SSC at 42°C, or 0.5X SSC, 0.1% SDS at 65°C. Highly stringent wash conditions include, for example, 0.2X SSC at 65°C.

*Preparation of NIP 45 V Polynucleotides*

A naturally occurring NIP 45 V1 polynucleotides can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated NIP 45 V1 polynucleotides. For example, restriction enzymes and probes can be used to isolate polynucleotide fragments which comprise NIP 45 V1 nucleotide sequences. Isolated polynucleotides are in preparations which are free or at least 70, 80, or 90% free of other molecules.

NIP 45 V1 cDNA molecules can be made with standard molecular biology techniques, using NIP 45 V1 mRNA as a template. NIP 45 V1 cDNA molecules can thereafter be replicated using molecular biology techniques known in the art and disclosed in manuals such as Sambrook *et al.* (1989). An amplification technique, such as PCR, can be used to obtain additional copies of polynucleotides of the invention, using either human genomic DNA or cDNA as a template.

Alternatively, synthetic chemistry techniques can be used to synthesizes NIP 45 V1 polynucleotides. The degeneracy of the genetic code allows alternate nucleotide sequences to be synthesized which will encode an NIP 45 V1 polypeptide having, for example, an amino acid sequence shown in SEQ ID NO.8 or a biologically active variant thereof.

*Extending NIP 45 V Polynucleotides*

Various PCR-based methods can be used to extend the nucleic acid sequences encoding the disclosed portions of human NIP 45 V1 polypeptide to detect upstream sequences such as promoters and regulatory elements. For example, restriction-site PCR uses universal primers to retrieve unknown sequence adjacent to a known locus (Sarkar, *PCR Methods Applic. 2,* 318-322, 1993). Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region (Triglia *et al., Nucleic Acids Res. 16,* 8186, 1988). Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA (Lagerstrom *et al., PCR Methods Applic. 1,* 111-119, 1991). In this method, multiple restriction enzyme digestions and ligations also can be used to place an engineered double-stranded sequence into an unknown fragment of the DNA molecule before performing PCR.

Another method which can be used to retrieve unknown sequences is that of Parker *et al., Nucleic Acids Res. 19,* 3055-3060, 1991). Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (e.g. GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

*Obtaining NIP 45 V1 Polypeptides*

NIP 45 V1 polypeptides can be obtained, for example, by purification from human cells, by expression of NIP 45 V1 polynucleotides, or by direct chemical synthesis.

*Protein Purification*

NIP 45 V1 polypeptides can be purified from any human cell which expresses the protein, including host cells which have been transfected with NIP 45 V1 polynucleotides. Thymus, spleen, lymph node, and other immune related tissues are particularly useful sources of NIP45 V1 polypeptides. A purified NIP45 V1 polypeptide is separated from other compounds which normally associate with the NIP 45 V1 polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

A preparation of purified NIP 45 V1 polypeptides is at least 80% pure; preferably, the preparations are 90%, 95%, or 99% pure. Purity of the preparations can be assessed by any means known in the art, such as SDS-polyacrylamide gel electrophoresis.

*Expression of NIP 45 V Polynucleotides*

To express an NIP 45 V1 polypeptide, an NIP 45 V1 polynucleotide can be inserted into an expression vector which contains the necessary elements for the transcription and translation of the inserted coding sequence. Methods which are well known to those skilled in the art can be used to construct expression vectors containing sequences encoding NIP 45 V1 polypeptides and appropriate transcriptional and translational control elements. These methods include *in vitro* recombinant DNA techniques, synthetic techniques, and *in vivo* genetic recombination. Such techniques are described, for example, in Sambrook *et al.* (1989) and in Ausubel *et al.,* CURRENT. PROTOCOLS IN MOLECULAR BIOLOGY, John Wiley & Sons, New York, N.Y., 1989.

A variety of expression vector/host systems can be utilized to contain and express sequences encoding an NIP 45 V1 polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e.g.*, baculovirus), plant cell systems transformed with virus expression vectors (*e.g.*, cauliflower mosaic virus, CaMV; tobacco mosaic virus, TMV) or with bacterial expression vectors (*e.g.,* Ti or pBR322 plasmids), or animal cell systems.

The control elements or regulatory sequences are those non-translated regions of the vector - enhancers, promoters, 5' and 3' untranslated regions - which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (e.g., heat shock, RUBISCO, and storage protein genes) or from plant viruses (e.g., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding an NIP 45 V1 polypeptide, vectors based on SV40 or EBV can be used with an appropriate selectable marker.

<u>*Bacterial and Yeast Expression Systems*</u>

In bacterial systems, a number of expression vectors can be selected depending upon the use intended for the NIP 45 V1 polypeptide. For example, when a large quantity of an NIP 45 V1 polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the NIP 45 V1 polypeptide can be ligated into the vector in frame with sequences for the amino-terminal Met and the subsequent 7 residues of β-galactosidase so that a hybrid protein is produced. pIN vectors (Van Heeke & Schuster, *J. Biol. Chem. 264,* 5503-5509, 1989) or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast *Saccharomyces cerevisiae,* a number of vectors containing constitutive or inducible promoters such as alpha factor, alcohol oxidase, and PGH can be used. For reviews, see Ausubel *et al.* (1989) and Grant *et al., Methods Enzymol. 153*, 516-544,1987.

<u>*Plant and Insect Expression Systems*</u>

If plant expression vectors are used, the expression of sequences encoding NIP 45 V1 polypeptides can be driven by any of a number of promoters. For example, viral promoters such as the 35S and 19S promoters of CaMV can be used alone or in combination with the omega leader sequence from TMV (Takamatsu, *EMBO J. 6,* 307-311, 1987). Alternatively, plant promoters such as the small subunit of RUBISCO or heat shock promoters can be used (Coruzzi *et al., EMBO J. 3*, 1671-1680, 1984; Broglie *et al., Science 224,* 838-843, 1984; Winter *et al., Results Probl. Cell Differ. 17,* 85-105, 1991). These constructs can be introduced into plant cells by direct DNA transformation or by pathogen-mediated transfection. Such techniques are described in a number of generally available reviews (*e.g.*, Hobbs or Murray, in McGRAW HILL YEARBOOK OF SCIENCE AND TECHNOLOGY, McGraw Hill, New York, N.Y., pp. 191-196, 1992).

An insect system also can be used to express an NIP 45 V1 polypeptide. For example, in one such system *Autographa californica* nuclear polyhedrosis virus (AcNPV) is used as a vector to express foreign genes in *Spodoptera frugiperda* cells or in *Trichoplusia* larvae. Sequences encoding NIP45 LIKE polypeptides can be cloned into a non-essential region of the virus, such as the polyhedrin gene, and placed under control of the polyhedrin promoter. Successful insertion of NIP 45 V1 polypeptides will render the polyhedrin gene inactive and produce recombinant virus lacking coat protein. The recombinant viruses can then be used to infect S. *frugiperda cells* or *Trichoplusia* larvae in which NIP 45 V1 polypeptides can be expressed (Engelhard *et al., Proc. Nat. Acad Sci. 91,* 3224-3227, 1994).

***Mammalian Expression Systems***

A number of viral-based expression systems can be used to express NIP45 V1 polypeptides in mammalian host cells. For example, if an adenovirus is used as an expression vector, sequences encoding NIP 45 V1 polypeptides can be ligated into an adenovirus transcription/translation complex comprising the late promoter and tripartite leader sequence. Insertion in a non-essential E1 or E3 region of the viral genome can be used to obtain a viable virus which is capable of expressing an NIP 45 V1 polypeptide in infected host cells (Logan & Shenk, *Proc. Natl. Acad. Sci. 81,* 3655-3659, 1984). If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (*e.g.*, liposomes, polycationic amino polymers, or vesicles).

Specific initiation signals also can be used to achieve more efficient translation of sequences encoding NIP 45 V1 polypeptides. Such signals include the ATG initiation codon and adjacent sequences. In cases where sequences encoding an NIP 45 V1 polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used (see Scharf *et al., Results Probl. Cell Differ. 20*, 125-162, 1994).

*Host Cells*

A host cell strain can be chosen for its ability to modulate the expression of the inserted sequences or to process the expressed NIP 45 V1 polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g.*, CHO, HeLa, MDCK, HEK293, B-lymphoma cells and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins: For example, cell lines which stably express NIP 45 V1 polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced NIP 45 V1 sequences. Resistant clones of stably transformed cells can be proliferated using tissue culture techniques appropriate to the cell type. See, for example, ANIMAL CELL CULTURE, R.I. Freshney, ed., 1986.

Any number of selection systems can be used to recover transformed cell lines. These include, but are not limited to, the herpes simplex virus thymidine kinase (Wigler *et al., Cell 11,* 223-32, 1977) and adenine phosphoribosyltransferase (Lowy *et al., Cell 22*, 817-23, 1980) genes which can be employed in *tk*$^-$ or *aprt*$^-$ cells, respectively. Also, antimetabolite, antibiotic, or herbicide resistance can be used as the basis for selection. For example, dhfr confers resistance to methotrexate (Wigler *et al., Proc, Natl. Acad Sci.* 77, 3567-70, 1980), *npt* confers resistance to the aminoglycosides, neomycin and G-418 (Colbere-Garapin *et al., J. Mol. Biol. 150,* 1-14, 1981), and *als.* and *pat* confer resistance to chlorsulfuron and phosphinotricin acetyltransferase, respectively (Murray, 1992, *supra*). Additional selectable genes have been described. For example, *trpB* allows cells to utilize indole in place of tryptophan, or *hisD,* which allows cells to utilize histinol in place of histidine (Hartman & Mulligan, *Proc. Natl. Acad. Sci. 85,* 8047-51, 1988). Visible markers such as anthocyanins, β-glucuronidase and its substrate GUS, and luciferase and its substrate luciferin, can be used to identify transformants and to quantify the amount of transient or stable protein expression attributable to a specific vector system (Rhodes *et al., Methods Mol. Biol. 55,* 121-131, 1995).

*Detecting Expression of NIP 45 V1 Polypeptides*

Although the presence of marker gene expression suggests that the NIP 45 V1 polynucleotide is also present, its presence and expression may need to be confirmed. For example, if a sequence encoding an NIP 45 V1 polypeptide is inserted within a marker gene sequence, transformed cells containing sequences which encode an NIP 45 V1 polypeptide can be identified by the absence of marker gene function. Alternatively, a marker gene can be placed in tandem with a sequence encoding an NIP45 V1 polypeptide under the control of a single promoter. Expression of the marker gene in response to induction or selection usually indicates expression of the NIP 45 V1 polynucleotide.

Alternatively, host cells which contain an NIP 45 V1 polynucleotide and which express an NIP 45 V1 polypeptide can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding an NIP 45 V1 polypeptide can be detected by DNA-DNA or DNA-RNA hybridization or amplification using probes or fragments or fragments of polynucleotides encoding an NIP 45 V1 polypeptide. Nucleic acid amplification-based assays involve the use of oligonucleotides selected from sequences encoding an NIP 45 V1 polypeptide to detect transformants which contain an NIP 45 V1 polynucleotide.

A variety of protocols for detecting and measuring the expression of an NIP 45 V1 polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on an NIP 45 V1 polypeptide can be used, or a competitive binding assay can be employed. These and other assays are described in Hampton *et al.,* SEROLOGICAL METHODS: A LABORATORY MANUAL, APS Press, St. Paul, Minn., 1990) and Maddox *et al., J. Exp. Med. 158*, 1211-1216, 1983).

A wide variety of labels and conjugation techniques are known by those skilled in the art and can be used in various nucleic acid and amino acid assays. Means for producing labeled hybridization or PCR probes for detecting sequences related to polynucleotides encoding NIP45 V1 polypeptides include oligolabeling, nick translation, end-labeling, or PCR amplification using a labeled nucleotide. Alternatively, sequences encoding an NIP 45 V1 polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of NIP 45 V1 Polypeptides*

Host cells transformed with nucleotide sequences encoding an NIP 45 V1 polypeptide can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing polynucleotides which encode NIP45 V1 polypeptides can be designed to contain signal sequences which direct secretion of soluble NIP 45 V1 polypeptides through a prokaryotic or eukaryotic cell membrane or which direct the membrane insertion of membrane-bound NIP 45 V1 polypeptide.

As discussed above, other constructions can be used to join a sequence encoding an NIP 45 V1 polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the NIP 45 V1 polypeptide also can be used to facilitate purification. One such expression vector provides for expression of a fusion protein containing an NIP 45 V1 polypeptide and 6 histidine residues preceding a thioredoxin or an enterokinase cleavage site. The histidine residues facilitate purification by IMAC (immobilized metal ion affinity chromatography, as described in Porath *et al., Prot. Exp. Purif. 3*, 263-281, 1992), while the enterokinase cleavage site provides a means for purifying the NIP 45 V1 polypeptide from the fusion protein. Vectors which contain fusion proteins are disclosed in Kroll *et al., DNA Cell Biol. 12,* 441-453, 1993.

*Chemical Synthesis*

Sequences encoding an NIP 45 V1 polypeptide can be synthesized, in whole or in part, using chemical methods well known in the art (see Caruthers *et al., Nucl. Acids Res. Symp. Ser.* 215-223, 1980; Horn *et al. Nucl. Acids Res. Symp. Ser.* 225-232, 1980). Alternatively, an NIP 45 V1 polypeptide itself can be produced using chemical methods to synthesize its amino acid sequence, such as by direct peptide synthesis using solid-phase techniques. (Merrifield, *J. Am. Chem. Soc. 85,* 2149-2154, 1963; Roberge *et al., Science 269,* 202-204, 1995). Protein synthesis can be performed using manual techniques or by automation. Automated synthesis can be achieved, for example, using Applied Biosystems 431 A Peptide Synthesizer (Perkin Elmer). Optionally, fragments of NIP 45 V1 polypeptides can be separately synthesized and combined using chemical methods to produce a full-length molecule.

The newly synthesized peptide can be substantially purified by preparative high performance liquid chromatography (*e.g.*, Creighton, PROTEINS: STRUCTURES AND MOLECULAR PRINCIPLES, WH Freeman and Co., New York, N.Y., 1983). The composition of a synthetic NIP 45 V1 polypeptide can be confirmed by amino acid analysis or sequencing (*e.g.*, the Edman degradation procedure; see Creighton, *supra*). Additionally, any portion of the amino acid sequence of the NIP 45 V1 polypeptide can be altered during direct synthesis and/or combined using chemical methods with sequences from other proteins to produce a variant polypeptide or a fusion protein.

*Production of Altered NIP 45 V Polypeptides*

As will be understood by those of skill in the art, it may be advantageous to produce NIP 45 V1 polypeptide-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter NIP 45 V1 polypeptide-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Antibodies*

Any type of antibody known in the art can be generated to bind specifically to an epitope of an NIP 45 V1 polypeptide. "Antibody" as used herein includes intact immunoglobulin molecules, as well as fragments thereof, such as Fab, $F(ab')_2$, and Fv, which are capable of binding an epitope of an NIP 45 V1 polypeptide. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to an epitope of an NIP 45 V1 polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to an NIP 45 V1 polypeptide provides a detection signal at least 5-, 10-, or 20-fold higher than a detection signal provided with other proteins when used in an immunochemical assay. Preferably, antibodies which specifically bind to NIP 45 V polypeptides do not detect other proteins in immunochemical assays and can immunoprecipitate an NIP 45 V1 polypeptide from solution.

NIP 45 V1 polypeptides can be used to immunize a mammal, such as a mouse, rat, rabbit, guinea pig, monkey, or human, to produce polyclonal antibodies. If desired, an NIP 45 V1 polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (*e.g.,* aluminum hydroxide), and surface active substances (*e.g.*, lysolecithin, pluronic polyols, polyanions, peptides, oil emulsions, keyhole limpet hemocyanin, and dinitrophenol). Among adjuvants used in humans, BCG *(bacilli Calmette-Guerin)* and *Corynebacterium parvum* are especially useful.

Monoclonal antibodies which specifically bind to an NIP 45 V1 polypeptide can be prepared using any technique which provides for the production of antibody molecules by continuous cell lines in culture. These techniques include, but are not limited to, the hybridoma technique, the human B-cell hybridoma technique, and the EBV-hybridoma technique (Kohler *et al., Nature 256*, 495-497, 1985; Kozbor *et al., J. Immunol. Methods 81,* 31-42, 1985; Cote *et al., Proc. Natl. Acad Sci. 80,* 2026-2030, 1983; Cole *et al., Mol. Cell Biol. 62*, 109-120, 1984).

In addition, techniques developed for the production of "chimeric antibodies," the splicing of mouse antibody genes to human antibody genes to obtain a molecule with appropriate antigen specificity and biological activity, can be used (Morrison *et al., Proc. Natl. Acad Sci. 81*, 6851-6855, 1984; Neuberger *et al., Nature 312,* 604-668, 1984; Takeda *et al., Nature 314,* 452-454, 1985). Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to an NIP 45 V1 polypeptide can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to NIP 45 V polypeptides. Antibodies with related specificity, but of distinct idiotypic composition, can be generated by chain shuffling from random combinatorial immunoglobin libraries (Burton, *Proc. Natl. Acad. Sci. 88*, 11120-23, 1991).

Single-chain antibodies also can be constructed using a DNA amplification method, such as PCR, using hybridoma cDNA as a template (Thirion *et al.,* 1996, *Eur. J Cancer Prev. 5*, 507-11). Single-chain antibodies can be mono- or bispecific, and can be bivalent or tetravalent. Construction of tetravalent, bispecific single-chain antibodies is taught, for example, in Coloma & Morrison, 1997, *Nat. Biotechnol. 15,* 159-63. Construction of bivalent, bispecific single-chain antibodies is taught in Mallender & Voss, 1994, *J. Biol. Chem. 269*, 199-206.

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology (Verhaar *et al.,* 1995, *Int. J. Cancer 61,* 497-501; Nicholls *et al.,* 1993, *J. Iminunol. Meth. 165,* 81-91).

Antibodies which specifically bind to NIP 45 V1 polypeptides also can be produced by inducing *in vivo* production in the lymphocyte population or by screening immunoglobulin libraries or panels of highly specific binding reagents as disclosed in the literature (Orlandi *et al., Proc. Natl. Acad Sci. 86*, 3833-3837, 1989; Winter *et al., Nature 349*, 293-299, 1991).

Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which an NIP 45 V1 polypeptide is bound. The bound antibodies can then be eluted from the column using a buffer with a high salt concentration.

## Antisense Oligonucleotides

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 of more nucleotides long. Longer sequences also can be used. Antisense oligonucleotide molecules can be provided in a DNA construct and introduced into a cell as described above to decrease the level of NIP 45 V1 gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters. *See* Brown, *Meth. Mol. BioL 20,* 1-8, 1994; Sonveaux, *Meth. Mol. Biot 26,* 1-72, 1994; Uhlmann *et al., Chem. Rev. 90,* 543-583, 1990.

Modifications of NIP 45 V1 gene expression can be obtained by designing antisense oligonucleotides which will form duplexes to the control, 5', or regulatory regions of the NIP 45 V1 gene. Oligonucleotides derived from the transcription initiation site, e.g., between positions -10 and +10 from the start site, are preferred. Similarly, inhibition can be achieved using "triple helix" base-pairirig methodology. Triple helix pairing is useful because it causes inhibition of the ability of the double helix to open sufficiently for the binding of polymerases, transcription factors, or chaperons.

Therapeutic advances using triplex DNA have been described in the literature (e.g., Gee *et al.*, in Huber & Carr, MOLECULAR AND IMMUNOLOGIC APPROACHES, Futura Publishing Co., Mt. Kisco, N.Y., 1994). An antisense oligonucleotide also can be designed to block translation of mRNA by preventing the transcript from binding to ribosomes.

Precise complementarity is not required for successful complex formation between an antisense oligonucleotide and the complementary sequence of an NIP 45 V1 polynucleotide. Antisense oligonucleotides which comprise, for example, 2, 3, 4, or 5 or more stretches of contiguous nucleotides which are precisely complementary to an NIP 45 V1 polynucleotide, each separated by a stretch of contiguous nucleotides which are not complementary to adjacent NIP 45 V1 nucleotides, can provide sufficient targeting specificity for NIP 45 V mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular NIP 45 V1 polynucleotide sequence.

Antisense oligonucleotides can be modified without affecting their ability to hybridize to an NIP 45 V1 polynucleotide. These modifications can be internal or at one or both ends of the antisense molecule. For example, intemucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art. *See, e.g.,* Agrawal *et al., Trends Biotechnol. 10,* 152-158, 1992; Uhlmann *et al., Chem. Rev. 90,* 543-584,1990; Uhlmann *et al:, Tetrahedron. Lett. 215,* 3539-3542, 1987.

*Ribozymes*

Ribozymes are RNA molecules with catalytic activity. *See, e.g.,* Cech, *Science 236,* 1532-1539; 1987; Cech, *Ann. Rev. Biochem. 59,* 543-568; 1990, Cech, *Curr. Opin. Struct. Biol.* 2, 605-609; 1992, Couture & Stinchcomb, *Trends Genet. 12,* 510-515, 1996. Ribozymes can be used to inhibit gene function by cleaving an RNA sequence, as is known in the art (*e.g.*, Haseloff *et al.,* U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The coding sequence of an NIP45 V1 polynucleotide, such as the nucleotide sequence shown in SEQ ID NO. 2, can be used to generate ribozymes which will specifically bind to mRNA transcribed from the NIP 45 V polynucleotide. Methods of designing and constructing ribozymes which can cleave other RNA molecules in trans in a highly sequence specific manner have been developed and described in the art (*see* Haseloff *et al. Nature 334,* 585-591, 1988). For example, the cleavage activity of ribozymes can be targeted to specific RNAs by engineering a discrete "hybridization" region into the ribozyme. The hybridization region contains a sequence complementary to the target RNA and thus specifically hybridizes with the target (see, for example, Gerlach *et al.,* EP 321,201).

Specific ribozyme cleavage sites within an NIP 45 V1 RNA target can be identified by scanning the target molecule for ribozyme cleavage sites which include the following sequences: GUA; GUU, and GUC. Once identified, short RNA sequences of between 15 and 20 ribonucleotides corresponding to the region of the target RNA containing the cleavage site can be evaluated for secondary structural features which may render the target inoperable. Suitability of candidate NIP45 V1 RNA targets also can be evaluated by testing accessibility to hybridization with complementary oligonucleotides using ribonuclease protection assays. The nucleotide sequences shown in SEQ ID NO.2 and their complements provide a source of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease NIP 45 V1 expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff *et al.,* U.S. Patent 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

*Screening Methods*

The invention provides assays for screening test compounds which bind to or modulate the activity of an NIP 45 V1 polypeptide or an NIP 45 V1 polynucleotide. A test compound preferably binds to an NIP 45 V1 polypeptide or polynucleotide. More preferably, a test compound decreases or increases the effect of NIP45 or an NIP45 analog as mediated via human NIP 45 V1 by at least about 10, preferably about 50, more preferably about 75, 90, or 100% relative to the absence of the test compound.

*Test Compounds*

Test compounds can be pharmacological agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead, one-compound" library method, and synthetic library methods using affinity chromatography selection. The biological library approach is limited to polypeptide libraries, while the other four approaches are applicable to polypeptide, non-peptide oligomer, or small molecule libraries of compounds. *See* Lam, *Anticancer Drug Des. 12,* 145, 1997.

Methods for the synthesis of molecular libraries are well known in the art *(see,* for example, DeWitt *et al., Proc. Natl. Acad Sci. U.S.A. 90,* 6909, 1993; Erb *et al. Proc. Natl. Acad Sci. U.S.A. 91,* 11422, 1994; Zuckermann *et al., J. Med Chem. 37*,2678, 1994; Cho *et al., Science 261,* 1303, 1993; Carell *et al., Angew. Chem. Int. Ed Engl. 33*, 2059, 1994; Carell *et al., Angew. Chem. Int. Ed. Engl. 33*, 2061; Gallop *et al., J. Med. Chem. 37,* 1233, 1994). Libraries of compounds can be presented in solution *(see, e.g.,* Houghten, *Biotechniques 13,* 412-421, 1992), or on beads (Lam, *Nature 354*, 82-84, 1991), chips (Fodor, *Nature 364,* 555-556, 1993), bacteria or spores (Ladner, U.S. Patent 5,223,409), plasmids (Cull *et al., Proc. Natl. Acad Sci. U.S.A.. 89*, 1865-1869, 1992), or phage (Scott & Smith, *Science 249,* 386-390, 1990; Devlin, *Science 249*, 404-406, 1990); Cwirla *et al., Proc. Natl. Acad Sci. 97,* 6378-6382, 1990; Felici, *J. Mol. Biol. 222*, 301-310, 1991; and Ladner, U.S. Patent 5,223,409).

*High Throughput Screening*

Test compounds can be screened for the ability to bind to NIP 45 V1 polypeptides or polynucleotides or to affect NIP 45 V1 activity or NIP 45 V1 gene expression using high throughput screening. Using high throughput screening, many discrete compounds can be tested in parallel so that large numbers of test compounds can be quickly screened. The most widely established techniques utilize 96-well microtiter plates. The wells of the microtiter plates typically require assay volumes that range from 50 to 500 µl. In addition to the plates, many instruments, materials, pipettors, robotics, plate washers, and plate readers are commercially available to fit the 96-well format.

Alternatively, "free format assays," or assays that have no physical barrier between samples, can be used. For example, an assay using pigment cells (melanocytes) in a simple homogeneous assay for combinatorial peptide libraries is described by Jayawickreme *et al*., *Proc. Natl. Acad. Sci. U.S.A. 19*, 1614-18 (1994). The cells are placed under agarose in petri dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is described by Chelsky, "Strategies for Screening Combinatorial Libraries: Novel and Traditional Approaches," reported at the First Annual Conference of The Society for Biomolecular Screening in Philadelphia, Pa. (Nov. 7-10, 1995). Chelsky placed a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV-light. Compounds that inhibited the enzyme were observed as local zones of inhibition having less color change.

Yet another example is described by *Salmon et al., Molecular Diversity 2*, 57-63 (1996). In this example, combinatorial libraries were screened for compounds that had cytotoxic effects on cancer cells growing in agar.

Another high throughput screening method is described in Beutel *et al.,* U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule which binds to and occupies the active site of the NIP 45 V1 polypeptide, thereby making the active site inaccessible or accessible to substrate (e.g., NFATp) such that normal biological activity is prevented. Examples of such small molecules include, but are not limited to, small peptides or peptide-like molecules.

In binding assays, either the test compound or the NIP 45 V1 polypeptide can comprise a detectable label, such as a fluorescent, radioisotopic, chemiluminescent, or enzymatic label, such as horseradish peroxidase, alkaline phosphatase, or luciferase. Detection of a test compound which is bound to the NIP 45 V1 polypeptide can then be accomplished, for example, by direct counting of radio-emmission, by scintillation counting, or by determining conversion of an appropriate substrate to a detectable product.

Alternatively, binding of a test compound to an NIP 45 V1 polypeptide can be determined without labeling either of the interactants. For example, a microphysiometer can be used to detect binding of a test compound with an NIP 45 V1 polypeptide. A microphysiometer (*e.g.*, Cytosensor™) is an analytical instrument that measures the rate at which a cell acidifies its environment using a light-addressable potentiometric sensor (LAPS). Changes in this acidification rate can be used as an indicator of the interaction between a test compound and an NIP 45 V1 polypeptide (McConnell *et al., Science 257*, 1906-1912, 1992).

Determining the ability of a test compound to bind to an NIP 45 V1 polypeptide also can be accomplished using a technology such as real-time Bimolecular Interaction Analysis (BIA) (Sjolander & Urbaniczky, *Anal. Chem. 63,* 2338-2345, 1991, and Szabo *et al., Curr. Opin. Struct. Biol. 5*, 699-705, 1995). BIA is a technology for studying biospecific interactions in real time, without labeling any of the interactants (*e.g.*, BIAcore™). Changes in the optical phenomenon surface plasmon resonance (SPR) can be used as an indication of real-time reactions between biological molecules.

In yet another aspect of the invention, an NIP 45 V1 polypeptide can be used as a "bait protein" in a two-hybrid assay or three-hybrid assay (see, *e.g.,* U.S. Patent 5,283,317; Zervos *et al., Cell 72*, 223-232, 1993; Madura *et al., J. Biol. Chem. 268,* 12046-12054, 1993; Bartel *et al., Biotechniques 14,* 920-924, 1993; Iwabuchi *et al., Oncogene 8,* 1693-1696, 1993; and Brent WO94/10300), to identify other proteins which bind to or interact with the NIP 45 V polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding an NIP45 V1 polypeptide can be fused to a polynucleotide encoding the DNA binding domain of a known transcription factor (*e.g.*, GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (*e.g.*, LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the NIP 45 V1 polypeptide.

It may be desirable to immobilize either the NIP 45 V1 polypeptide (or polynucleotide) or the test compound to facilitate separation of bound from unbound forms of one or both of the interactants, as well as to accommodate automation of the assay. Thus, either the NIP 45 V1 polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach the NIP 45 V1 polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to an NIP 45 V1 polypeptide (or polynucleotide) can be accomplished in any vessel suitable for containing the reactants. Examples of such vessels include microtiter plates, test tubes, and microcentrifuge tubes.

In one embodiment, the NIP 45 V1 polypeptide is a fusion protein comprising a domain that allows the NIP 45 V1 polypeptide to be bound to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed NIP 45 V1 polypeptide; the mixture is then incubated under conditions conducive to complex formation (*e.g.,* at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either an NIP 45 V1 polypeptide (or polynucleotide) or a test compound can be immobilized utilizing conjugation of biotin and streptavidin. Biotinylated NIP 45 V1 polypeptides (or, polynucleotides) or test compounds can be prepared from biotin-NHS (N-hydroxysuccinimide) using techniques well known in the art (*e.g.,* biotinylation kit. Pierce Chemicals, Rockford, Ill.) and immobilized in the wells of streptavidin-coated 96 - well plates (Pierce Chemical). Alternatively, antibodies which specifically bind to an NIP 45 V1 polypeptide, polynucleotide, or a test compound, but which do not interfere with a desired binding site, such as the active site of the NIP 45 V1 polypeptide, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to the NIP 45 V1 polypeptide or test compound, enzyme-linked assays which rely on detecting an activity of the NIP 45 V1 polypeptide, and SDS gel electrophoresis under non-reducing conditions.

Screening for test compounds which bind to an. NIP45 V1 polypeptide or polynucleotide also can be carried out in an intact cell. Any cell which comprises an NIP45 V polypeptide or polynucleotide can be used in a cell-based assay system. An NIP 45 V polynucleotide can be naturally occurring in the cell or can be introduced using techniques such as those described above. Binding of the test compound to an NIP 45 V polypeptide or polynucleotide is determined as described above.

*Functional Assays*

Test compounds can be tested for the ability to increase or decrease a biological effect of an NIP 45 V1 polypeptide. Such biological effects can be determined using the functional assays described in the specific examples, below. Functional assays can be carried out after contacting either a purified NIP45 V1 polypeptide, a cell membrane preparation, or an intact cell with a test compound. A test compound which decreases a functional activity of an NIP 45 V1 by at least about 10, preferably about 50, more preferably about 75, 90, or 100% is identified as a potential agent for decreasing NIP 45 Vfactivity. A test compound which increases NIP 45 V1 activity by at least about 10, preferably about 50, more preferably about 75, 90, or 100% is identified as a potential agent for increasing NIP 45 V1 activity.

One such screening procedure involves the use of B-lymphoma cells which are transfected to express an NIP 45 V1 polypeptide. For example, such an assay may be employed for screening for a compound which inhibits activation of the polypeptide by exposing the transfected B-lymphoma cells which comprise the polypeptide with both endogenously interacting proteins or substrates to a test compound to be screened. Inhibition of the activity of the polypeptide indicates that a test compound is a potential antagonist for the polypeptide, i.e., inhibits the function of the protein. The screen may be employed for identifying a test compound which activates the protein by exposing such cells to compounds to be screened and determining whether each test compound activates the protein.

Other screening techniques include the use of cells which express a human NIP 45 V1 polypeptide (for example, transfected T cells) in a system which measures amounts of secreted proteins generated by polypeptide activation. For example, test compounds may be added to cells that express a human NIP45 V1 polypeptide and the expression of a reporter gene with specific promoter sequences can be measured to determine whether the test compound activates or inhibits the protein.

Details of functional assays, such as those described above, are provided in the specific examples below.

*NIP 45 V Gene Expression*

In another embodiment, test compounds which increase or decrease NIP 45 V1 gene expression are identified. An NIP 45 V1 polynucleotide is contacted with a test compound, and the expression of an RNA or polypeptide product of the NIP 45 V1 polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of NIP 45 V1 mRNA or polypeptide expression in the cells can be determined by methods well known in the art for detecting mRNA or polypeptide. Either qualitative or quantitative methods can be used. The presence of polypeptide products of an NIP 45 V1 polynucleotide can be determined, for example, using a variety of techniques known in the art, including immunochemical methods such as radioimmunoassay, Western blotting, and immunohistochemistry. Alternatively, polypeptide synthesis can be determined *in vivo*, in a cell culture, or in an *in vitro* translation system by detecting incorporation of labeled amino acids into an NIP 45 V1 polypeptide.

Such screening can be carried out either in a cell-free assay system or in an intact cell. Any cell which expresses an NIP 45 V1 polynucleotide can be used in a cell-based assay system. The NIP 45 V1 polynucleotide can be naturally occurring in the cell or can be introduced using techniques such as those described above. Either a primary culture or an established cell line, such as CHO or human embryonic kidney 293 cells, can be used.

*Pharmaceutical Compositions*

The invention also provides pharmaceutical compositions which can be administered to a patient to achieve a therapeutic effect. Pharmaceutical compositions of the invention can comprise, for example, an NIP 45 V1 polypeptide, NIP 45 V1 polynucleotide, antibodies which specifically bind to an NIP 45 V1 polypeptide, or mimetics, enhancers and inhibitors, or inhibitors of an NIP45 V1 polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically-acceptable carriers comprising excipients and auxiliaries which facilitate processing of the active compounds into preparations which can be used pharmaceutically. Pharmaceutical compositions of the invention can be administered by any number of routes including, but not limited to, oral, intravenous, intramuscular, intra-arterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, *i.e.*, dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents which increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions of the present invention can be manufactured in a manner that is known' in the art, *e.g.*, by means of conventional mixing, dissolving, granulating, dragee-making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilized powder which can contain any or all of the following: 1-50 mM histidine, 0.1%-2% sucrose, and 2-7% mannitol, at a pH range of 4.5 to 5.5, that is combined with buffer prior to use.

Further details on techniques for formulation and administration can be found in the latest edition of REMINGTON'S PHARMACEUTICAL SCIENCES (Maack Publishing Co., Easton, Pa.). After pharmaceutical compositions have been prepared, they can be placed in an appropriate container and labeled for treatment of an indicated condition. Such labeling would include amount, frequency, and method of administration.

*Therapeutic Indications and Methods*

NIP 45 variant of the present invention is responsible for many biological functions, including many pathologies. Accordingly, it is desirable to find compounds and drugs which stimulate NIP 45 variant on the one hand and which can inhibit the function of a NIP 45 variant on the other hand. Compounds which can modulate the function or expression of NIP 45 variant are useful in treating various allergic diseases, autoimmune diseases, inflammatory deseases, and infectious deseases including asthma, allergic rhinitis, atopic dermatitis, hives, conjunctivitis, vernal catarrh, chronic arthrorheumatism, systemic lupus erythematosus, myasthenia gravis, psoriasis, diabrotic colitis, systemic inflammatory response syndrome (SIRS), llymphofollicular thymitis, sepsis, polymyositis, dermatomyositis, polyaritis nodoa, mixed connective tissue disease (MCTD), Sjoegren's syndrome, gout, and the like.

This invention further pertains to the use of novel agents identified by the screening assays described above. Accordingly, it is within the scope of this invention to use a test compound identified as described herein in an appropriate animal model. For example, an agent identified as described herein (*e.g.,* a modulating agent, an antisense nucleic acid molecule, a specific antibody, ribozyme, or an NIP 45 V1 polypeptide binding molecule) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

A reagent which affects NIP45 variant activity can be administered to a human cell, either *in vitro* or *in vivo,* to reduce NIP 45 V1 like activity. The reagent preferably binds to an expression product of a human NIP45 variant gene. If the expression product is a protein, the reagent is preferably an antibody. For treatment of human cells *ex vivo,* an antibody can be added to a preparation of stem cells which have been removed from the body. The cells can then be replaced in the same or another human body, with or without clonal propagation, as is known in the art.

In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful in the present invention comprises a lipid composition that is capable of fusing with the plasma membrane of the targeted cell to deliver its contents to the cell. Preferably, the transfection efficiency of a liposome is about 0.5μg of DNA per 16 nmole of liposome delivered to about $10^6$ cells, more preferably about 1.0 μg of DNA per 16 nmole of liposome delivered to about $10^6$ cells, and even more preferably about 2.0 μg of DNA per 16 nmol of liposome delivered to about $10^6$ cells. Preferably, a liposome is between about 100 and 500 nm, more preferably between about 150 and 450 nm, and even more preferably between about 200 and 400 nm in diameter.

Suitable liposomes for use in the present invention include those liposomes standardly used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a tumor cell, such as a tumor cell ligand exposed on the outer surface of the liposome.

Complexing a liposome with a reagent such as an antisense oligonucleotide or ribozyme can be achieved using methods which are standard in the art (see, for example, U.S. Patent 5,705,151). Preferably, from about 0.1 μg to about 10 μg of polynucleotide is combined with about 8 nmol of liposomes, more preferably from about 0.5 μg to about 5 μg of polynucleotides are combined with about 8 nmol liposomes, and even more preferably about 1.0 μg of polynucleotides is combined with about 8 nmol liposomes.

In another embodiment, antibodies can be delivered to specific tissues *in vivo* using protein-mediated targeted delivery. Protein-mediated DNA delivery techniques are taught in, for example, Findeis *et al. Trends in Biotechnol. 11,* 202-05 (1993); Chiou *et al.,* GENE THERAPEUTICS: METHODS AND APPLICATIONS OF DIRECT GENE TRANSFER (J.A. Wolff, ed.) (1994); Wu & Wu, *J. Biol. Chem. 263*, 621-24 (1988); Wu *et al., J. Biol. Chem. 269,* 542-46 (1994); Zenke *et al., Proc. Natl. Acad Sci. U.S.A. 87*, 3655-59 (1990); Wu *et al., J. Biol. Chem. 266*, 338-42 (1991).

*Determination of a Therapeutically Effective Dose*

The determination of a therapeutically effective dose is well within the capability of those skilled in the art. A therapeutically effective dose refers to that amount of active ingredient which increases or decreases NIP 45 V1 activity relative to the NIP 45 V1 activity that occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, *e.g.*, $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun," and DEAE- or calcium phosphate-mediated transfection.

Effective *in vivo* dosages of an antibody are in the range of about 5 μg to about 50 μg/kg, about 50 μg to about 5 mg/kg, about 100 μg to about 500 μg/kg of patient body weight, and about 200 to about 250 μg/kg of patient body weight. For administration of polynucleotides encoding single-chain antibodies, effective *in* vivo dosages are in the range of about 100 ng to about 200 ng, 500 ng to about 50 mg, about 1 μg to about 2 mg, about 5 μg to about. 500 μg, and about 20 μg to about 100 μg of DNA.

If the expression product is mRNA, the reagent is preferably an antisense oligonucleotide or a ribozyme. Polynucleotides which express antisense oligo-nucleotides or ribozymes can be introduced into cells by a variety of methods, as described above.

Preferably, a reagent reduces expression of an NIP 45 V1 gene or the activity of an NIP 45 V1 polypeptide by at least about 10, preferably about 50, more preferably about 75, 90, or 100% relative to the absence of the reagent. The effectiveness of the mechanism chosen to decrease the level of expression of an NIP 45 V1 gene or the activity of an NIP 45 V1 polypeptide can be assessed using methods well known in the art, such as hybridization of nucleotide probes to NIP 45 V1-specific mRNA, quantitative RT-PCR, immunologic detection of an NIP 45 V1 polypeptide, or measurement of NIP 45 V1 activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, mammals such as dogs, cats, cows, horses, rabbits, monkeys, and most preferably, humans.

*Diagnostic Methods*

NIP 45 variant also can be used in diagnostic assays for detecting diseases and abnormalities or susceptibility to diseases and abnormalities related to the presence of mutations in the nucleic acid sequences which encode a NIP 45 variant. Such diseases, by way of example, are related to various allergic diseases, autoimmune diseases, inflammatory deseases, and infectious deseases including asthma, allergic rhinitis, atopic dermatitis, hives, conjunctivitis, vernal catarrh, chronic arthrorheumatism, systemic lupus erythematosus, myasthenia gravis, psoriasis, diabrotic colitis, systemic inflammatory response syndrome (SIRS), llymphofollicular thymitis, sepsis, polymyositis, dermatomyositis, polyaritis nodoa, mixed connective tissue disease (MCTD), Sjoegren's syndrome, gout, and the like.

Differences can be determined between the cDNA or genomic sequence encoding a NIP 45 variant in individuals afflicted with a disease and in normal individuals. If a mutation is observed in some or all of the afflicted individuals but not in normal individuals, then the mutation is like ly to be the causative agent of the disease.

Sequence differences between a reference gene and a gene having mutations can be revealed by the direct DNA sequencing method. In addition, cloned DNA segments can be employed as probes to detect specific DNA segments. The sensitivity of this method is greatly enhanced when combined with PCR. For example, a sequencing primer can be used with a double-stranded PCR product or a single-stranded template molecule generated by a modified PCR. The sequence determination is performed by conventional procedures using radiolabeled nucleotides or by automatic sequencing procedures using fluorescent tags.

Genetic testing based on DNA sequence differences can be carried out by detection of alteration in electrophoretic mobility of DNA fragments in gels with or without denaturing agents. Small sequence deletions and insertions can be visualized, for example, by high resolution gel electrophoresis. DNA fragments of different sequences can be distinguished on denaturing formamide gradient gels in which the mobilities of different DNA fragments are retarded in the gel at different positions according to their specific melting or partial melting temperatures (*see, e.g.,* Myers *et al., Science 230,* 1242, 1985). Sequence changes at specific locations can also be revealed by nuclease protection assays, such as RNase and S 1 protection or the chemical cleavage method (e.g., Cotton *et al., Proc. Natl. Acad. Sci. USA 85*, 4397-4401, 1985). Thus, the detection of a specific DNA sequence can be performed by methods such as hybridization, RNase protection, chemical cleavage, direct DNA sequencing or the use of restriction enzymes and Southern blotting of genomic DNA. In addition to direct methods such as gel-electrophoresis and DNA sequencing, mutations can also be detected by *in situ* analysis.

Altered levels of a NIP 45 variant also can be detected in various tissues. Assays used to detect levels of the protein polypeptides in a body sample, such as blood or a tissue biopsy, derived from a host are well known to those of skill in the art and include radioimmunoassays, competitive binding assays, Western blot analysis, and ELISA assays.

The above disclosure generally describes the present invention. A more complete understanding can be obtained by reference to the following specific examples which are provided for purposes of illustration only and are not intended to limit the scope of the invention.

EXAMPLE 1

The murine NIP45 mRNA sequence (GenBank accession number U76759) was used to search the EST subset of the DNA DataBank of Japan (DDBJ) for homologous sequences using the computer program BLAST 2.0 (National Center for Biotechnology Information). Five sequences were found that had over 75% nucleotide sequence identity with the murine NIP45 mRNA sequence and these were selected as potential fragments of the human NIP45 mRNA sequence. The five sequences were GenBank accession numbers AI954626, AA919081, AA063239, AA351060, and AA352196. To gather further sequence information on the potential human MP45 mRNA sequence, the five sequences were themselves used to search the DDBJ for homologous sequences. As a result of the second search, an additional EST, GenBank accession number T56384, was identified. A third search with the T56384 EST was then performed and identified one additional EST, GenBank accession number W31117.

Clones containing four of the ESTs were purchased from Genome Systems (St. Louis, MO, USA) for further sequence evaluation. The full inserts of EST clones AA919081, AA063239, T56384, and W31117 were sequenced on a ABI Prism 377 DNA sequencer (PE Biosystems) according to the manufacturer's standard sequencing protocol using primers complemetary to the T3 and T7 promoter regions flanking the insert on each vector. After full sequences were obtained, the sequences of all seven ESTs were aligned using the computer program Sequencher (GeneCodes Corporation, Ann Arbor, MI, USA), forming a contiguous sequence of DNA. The consensus sequence of this contig was considered to represent the human NIP45 mRNA sequence. Nucleotide sequences of human NIP 45 is depicted in SEQ ID NO. 6. The open reading frame of the native human homologue of NIP 45 is composed of 1260 nucleotides (SEQ ID NO.7).

EXAMPLE 2

Identification of the alternative splice variants of NIP45

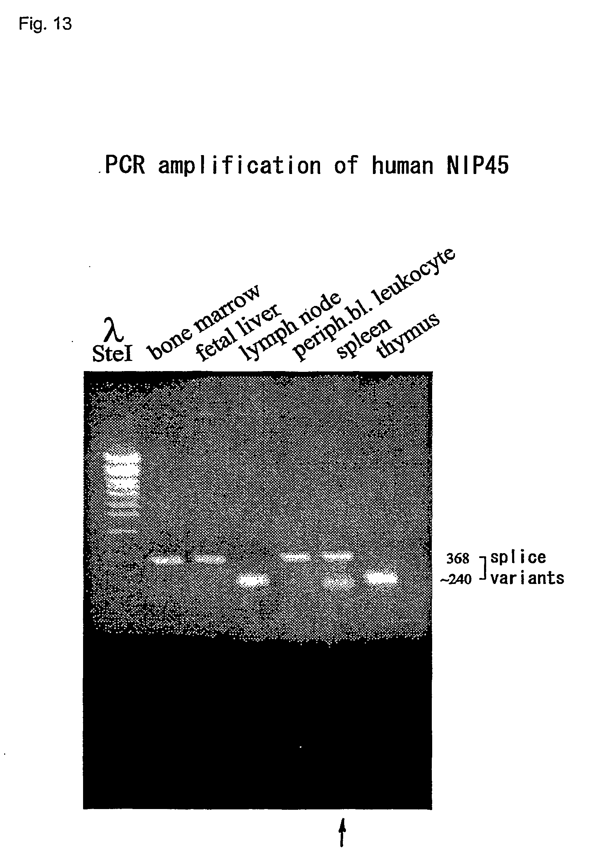

To test for expression of NIP45 in various tissue types, PCR amplification of a 368 base pair region of the human NIP45 cDNA was carried out using the oligonucleotide primers NIP-11 (cccgactcttcccactcaaaatcc, corresponding to 797-820 position of the sequence depicted in SEQ ID NO.6) and NIP12 (cagtcccatggcctcctcatagtg, corresponding to 1141-1164 position of the sequence depicted in SEQ ID NO.6) and the Human Immune System Multiple Tissue cDNA Panel (Clontech Laboratories, Inc, Palo Alto, CA, USA) as template. Amplification was performed with AmpliTaq Gold DNA polymerase (Perkin-Elmer) under the following PCR cycling conditions: Denaturation of template DNA for 9 minutes at 96°C followed by a cycling between 96°C for 15 seconds and 66°C for 30 seconds repeated 55 times. As shown in Fig. 13, PCR amplification of NIP45 cDNA derived from bone marrow, fetal liver, and peripheral blood leukocytes gave the expected 368 base pair product, whereas PCR amplification of NIP45 cDNA derived from lymph node and thymus gave a much shorter product and PCR amplification of NIP45 cDNA derived from spleen gave both the 368 base pair product and the shorter product.

Direct sequencing of the shorter PCR product were carried out on a ABI Prism 377 DNA sequencer (PE Biosystems) as described in Example I except using primers NIP-11 and NIP12. The shorter length of the product was due to the absence of a stretch of 255 base pairs compared to the NIP45 DNA sequence. Alignment of the shorter PCR product with the NIP45 DNA sequence and a genomic sequence fragment derived from the NIP45 gene indicated that the boundaries of the 255 bp of deleted sequence correspond exactly with the boundaries of two exons contained within the genomic sequence fragment. This splice variant was designated NIP45v1. Nucleotide sequences of cDNA prepared from mRNAs of human NIP 45V1 is depicted in SEQ ID NO.1.

Subsequent PCR amplification of the full coding region of NIP45 using spleen cDNA from the above panel as template and oligonucleotide primers NIP-L4 (aaagtgtgccatggcggagcatgt, corresponding to position 3-26 of the sequence depicted in SEQ ID NO.6) and NIP-R4 (gggtgtcagccccagacctcaat, corresponding to position 1255-1277 of SEQ ID NO. 6) produced several differently sized amplimers. The amplimers were cloned into the cloning vector pCRII-TOPO (Invitrogen Corp., Carlsbad, CA, USA) and sequenced on an ABI Prism 377 DNA sequencer (Applied Biosystems, Foster City, CA, USA). Analysis of the sequences obtained revealed three additional alternative splice variants, designated NIP45v2, NIP45v3, and MP4Sv4, respectively.

Properties of the NIP45v1 cDNA nucleotide sequence (SEQ ID NO.1 and 2)

1. Deletion of 255 base pairs corresponding to bases 847 through 1101 of the coding sequence of the full NIP45 transcript.

2. Deleted region corresponds precisely to two exons when compared with a partial genomic sequence of the NIP45 gene (clone RPCI-11-449D23, genomic survey sequence, Accession number AQ584348), indicating that the deletion is due to an alternative splicing event.

Properties of the NIP45v1 amino acid sequence (SEQ ID NO.8)

1. Conceptual translation gives a protein of 334 amino acids in length, 85 amino acid residues shorter than the full length NIP45 protein of 419 amino acids.

2. Amino acid residues missing in the conceptual translation of NIP45v1 correspond to amino acid residues 283 through 367 of the full-length NIP45 protein.

Properties of the spliced-out regions

The NIP45 protein sequence has significant homology to UBL1 and SMT3H2, ubiquitin-like proteins also known as Sentrin and Sentrin2. This Sentrin-homologous domain of NIP45 extends from amino acid residues 342 to 418 and contains 44 of 77 residues (56%) that are similar to the SMT3H2 sequence, among which 28 of 77 residues (36%) are identical to the SMT3H2 sequence as identified by the BLAST sequence similarity searching software (National Center for Biotechnology Information). NIP45 also has a region of homology with Ubiquitin from amino residues 274 to 334, showing an 84.7% alignment against a position specifc scoring matrix for Ubiquitin homologs in the Conserved Domain Database of the National Center for Biotechnology Information. The Sentrin-homologous domain is partially deleted in NIP45v1. The Ubiquitinhomologous domain is 85% deleted in NIP45v1.

Tissue distribution of NIP45v1

The NIP45v1 splice variant of NIP45 has only been found to be expressed in thymus, spleen, and lymph node, whereas NIP45 expression has been found in all immune related tissues tested to date, including bone marrow, fetal liver, lymph node, peripheral blood leukocytes, spleen, thymus, and tonsil.

EXAMPLE 3

Functional Characterization

The functions of NIP 45 variants are assessed by their ability of specifically interacting with NF-ATp in mammalian cells. Each of the NIP 45 V cDNA inserts (V1-V4) obtained in Example 2 is subcloned into a mammalian expression vector which fuses the coding region to an epitope tag from a influenza hemagglutinin (HA) peptide, vector pCEP4-HA (Herrscher, R. F. et al. (1995) Genes Dev. 9:3067-3082), to create the expression vector.

The vector is then cotransfected with an NF-ATp expression plasmid into HepG2 cells expressing low levels of NF-ATp. As controls, HepG2 cells also are cotransfected with NIP45-HA along with the expression vector without the NF-ATp insert or with the NF-ATp expression vector along with an out of frame fusion of NIP45 with the epitope tag. Lysates are prepared from the transfected cells and immunoprecipitated with anti-NF-ATp antibody. Western blot analysis are then performed on the immunprecipitated material using either anti-NF-ATp or anti-HA antibodies and it is seen that NF-AT and NIP 45 variant physically associate in vivo in mammalian cells.

EXAMPLE 4

Tissue Expression of NIP 45 V2-V4 mRNA

Quantitative reverse transcription-polymerase chain reaction (RT-PCR) analysis of RNA from different human tissues is performed to investigate the tissue expression of NIP 45 V2-4 mRNA. 100 .mu.g of total RNA from various tissues (Human Total RNA Panel I-V, Clontech Laboratories, Palo Alto, CA, USA) is used as a template to synthsize first-strand cDNA using the SUPERSCRIPT™ First-Strand Syntheswas System for RT-PCR (Life Technologies, Rockville , MD, USA). 10 ng of the first-strand cDNA is then used as template in a polymerase chain reaction to test for the presence of the NIP 45 V mRNA transcript. The polymerase chain reaction is performed in a LightCycler (Roche Molecular Biochemicals, Indianapolis, IN, USA), in the presence of the DNA-binding fluorescent dye SYBR Green I which binds to the minor groove of the DNA double helix, produced only when double-stranded DNA is successfully synthesized in the reaction, and upon binding, emits light that can be quantitatively measured by the LightCycler machine. The polymerase chain reaction is carried out using oligonucleotide primers designed to span the junction of spliced exons flanking deleted regions and measurements of the intensity of emitted light are taken following each cycle of the reaction when the reaction reach a temperature of 86 degrees C. Intensities of emitted light are converted into copy numbers of the gene transcript per nanogram of template cDNA by comparison with simultaneously reacted standards of known concentration.

To correct for differences in mRNA transcription levels per cell in the various tissue types, a normalization procedure is performed using calculated expression levels in the various tissues of five different housekeeping genes: glyceraldehyde-3-phosphatase (G3PHD), hypoxanthine guanine phophoribosyl transferase (HPRT), beta-actin, porphobilinogen deaminase (PBGD), and beta-2-microglobulin. Except for the use of a slightly different set of housekeeping genes, the normalization procedures is essentially the same as that described in the RNA Master Blot User Manual, Apendix C (Clontech Laboratories, Palo Alto, CA, USA).

EXAMPLE 5

Functional Activity of NIP45 variants in Regulating Gene Expression

To test for a functional role of NIP45 variants in NF-AT-driven transcription, each of the NIP45 Vs is expressed at high levels in HepG2 cells. HepG2 cells express low levels of endogenous NF-AT, and ectopic expression of NF-AT family member proteins has been shown to transactivate NF-AT-driven transcription in this cell line in the absence of exogenous stimulation (Hoey, T. et al. (1995) Immunity 2:461-472). HepG2 cells are transfected with a 3X NF-AT-CAT reporter gene (Venkataraman, L. et al. (1994) Immunity 1:189-196) and either control or expression plasmids for NIP45 variants and NF-AT family members (NF-ATp, NF-ATc, NF-AT3, NF-AT4). This reporter gene contains three tandem copies of the NF-AT binding site derived from the IL-2 gene. Alternatively, Hep G2 cells are transfected with an IL-4-CAT reporter construct (extending to -732 bp of the IL-4 promoter and containing a native NF-AT-dependent promoter) and expression vectors or controls for NIP45 variants, NF-ATp, and c-maf. HepG2 cells are transfected by the DEAE-Dextran method as described in Hoey, T. et al. (1995) supra, and CAT assays are performed according to standard methodologies.

EXAMPLE 6

Endogenous IL-4 Production

Expression of endogenous IL-4 by cells that do not normally produce IL-4 is examined to see if the combination of any of the NIP45 variants, NF-ATp and c-Maf is sufficient to induce the expression. M12 B lymphoma cells are transiently cotransfected with expression plasmids for NF-ATp and c-Maf together with NIP45 or pCI vector control. M12 cells are transiently transfected by electroporation as previously described (Ho, I. C. et al. (1996) Cell 85:973-983). Levels of IL-4 in the supernatants harvested 72 hours later are measured by a commercially available IL-4 ELISA (Pharmingen), performed according to the manufacturer's instructions except with modification as described (Ho, I. C. et al. (1996) supra).

EXAMPLE 7

Identification of a test compound which binds to a NIP 45 variant

The purified NIP 45 V1 polypeptide comprising a glutathione-S-transferase protein and absorbed onto glutathione-derivatized wells of 96-well microtiter plates is contacted with test compounds from a small molecule library at pH 7.0 in a physiological buffer solution. NIP 45 V1 polypeptides comprise any of the amino acid sequence shown in SEQ ID NO.8. The test compounds comprise a fluorescent tag. The samples are incubated for 5 minutes to one hour. Control samples are incubated in the absence of a test compound.

The buffer solution containing the test compounds is washed from the wells. Binding of a test compound to an NIP 45 V1 polypeptide is detected by fluorescence measurements of the contents of the wells. A test compound which increases the fluorescence in a well by at least 15% relative to fluorescence of a well in which a test compound is not incubated is identified as a compound which binds to an NIP 45 V1 polypeptide.

EXAMPLE 8

Identification of a test compound which modulates (increases or decreases) NIP 45 V1 gene expression

A test compound is administered to a culture of human lymph node cells and incubated at 37°C for 10 to 45 minutes. A culture of the same type of cells incubated for the same time without the test compound provides a negative control.

RNA is isolated from the two cultures as described in Chirgwin *et al., Biochem. 18,* 5294-99, 1979). Northern blots are prepared using 20 to 30 μg total RNA and hybridized with a $^{32}$P-labeled NIP 45 V1-specific probe at 65°C in Express-hyb (CLONTECH). The probe comprises at least 11 contiguous nucleotides selected from the complement of SEQ ID NO.1 or 2. A test compound which modulates the NIP 45 V1-specific signal relative to the signal obtained in the absence of the test compound is identified as an modulator of NIP 45 V1 gene expression.

EXAMPLE 9

Screening for a compound which modulates the interaction between NIP 45 variant and NF-AT can be done with the use of yeast two-hybrid system (s).

EXAMPLE 10

Treatment of immunologically related diseases by modulating the function of a human NIP 45 variant.

A polynucleotide which expresses a human NIP 45 variant or a compound, which modulate the function of NIP 45 variant is administered to a patient. The severity of the patient's inflammation is lessened.

## Claims

1. An isolated polynucleotide selected from the group consisting of

   i) polynucleotides encoding a polypeptide comprising an amino acid sequence shown in SEQ ID NO: 8 lacking amino acid residues 283 through 367 with respect to the full length NIP45 protein, which are able to interact with NFATp in order to potentiate transcription of the IL-4 gene;
   ii) polynucleotides comprising the sequence of SEQ ID NO: 2 lacking the sequence part coding for amino acid residues 283 through 367 with respect to the full length NIP45 protein, wherein the encoded proteins are able to interact with NFATp in order to potentiate transcription of the IL-4 gene.

2. An expression vector containing any polynucleotide of claim 1.

3. A host cell containing the expression vector of claim 2.

4. A substantially purified polypeptide encoded by a polynucleotide of claim 1.

5. A method for producing a polypeptide according to claim 4, wherein the method comprises the following steps:

   a. culturing the host cell of claim 3 under conditions suitable for the expression of the polypeptide according to claim 4; and
   b. recovering the polypeptide according to claim 4 from the host cell culture.

6. A method for detection of a polynucleotide according to claim 1 in a biological sample taken from a human or animal body comprising the following steps:

   a. hybridizing any polynucleotide of claim 1 or 2 to a nucleic acid material of a biological sample taken from a human or animal body, thereby forming a hybridization complex; and
   b. detecting said hybridization complex.

7. The method of claim 6, wherein before hybridization, the nucleic acid material of the biological sample is amplified.

8. A method for the detection of a polypeptide of claim 4 comprising the steps of:

   a. contacting a biological sample taken from a human or animal body with an antibody which specifically interacts with the polypeptide and
   b. detecting the interaction.

9. A diagnostic kit for conducting the method of any one of claims 6 to 8.

10. A method of screening for agents which decrease the activity of a polypeptide of claim 4, comprising the steps of:

   a. contacting a test compound with any polypeptide encoded by any polynucleotide of claim 1 or with any polynucleotide according to claim1;
   b. detecting binding of the test compound to the polypeptide encoded by any polynucleotide of claim 1 or to the polynucleotide of claim 1, wherein a test compound which binds to the polypeptide or to the polynucleotide is identified as a potential therapeutic agent for decreasing the activity of a NIP45 splice variant polypeptide according to claim 4.

**11.** A method of screening for agents which regulate the activity of a polypeptide of claim 4, comprising the steps of:

a. contacting a test compound with a polypeptide encoded by any polynucleotide of claim 1 or 2; and
b. detecting an activity of the polypeptide, wherein a test compound which increases the activity is identified as a potential therapeutic agent for increasing the activity of the polypeptide, and wherein a test compound which decreases the activity of the polypeptide is identified as a potential therapeutic agent for decreasing the activity of the polypeptide.

**12.** A pharmaceutical composition, comprising the expression vector of claim 2 and a pharmaceutically acceptable carrier.

**13.** Use of the expression vector of claim 2 in the preparation of a medicament for modulating the activity of a NIP45 splice variant polypeptide lacking amino acid residues 283 through 367 with respect to the full length NIP45 protein in a disease.

**14.** Use of claim 13 wherein the disease is an autoimmune, allergic, infectious or chronic inflammatory disease.

**15.** Use of claim 14 wherein the disease is asthma.

**Patentansprüche**

**1.** Isoliertes Polynucleotid, ausgewählt aus der aus Folgendem bestehenden Gruppe:

i) Polynucleotiden, die für ein Polypeptid kodieren, das eine in Seq.-ID Nr. 8 gezeigte Aminosäuresequenz aufweist, der im Vergleich mit dem Volllängen-NIP45-Protein die Aminosäurereste 283 bis 367 fehlen, die in der Lage sind, mit NFATp wechselzuwirken, um die Transkription des IL-4-Gens zu potenzieren;
ii) Polynucleotiden, welche die Sequenz von Seq.-ID Nr. 2 umfassen, welcher der Sequenzteil fehlt, der für die Aminosäurereste 283 bis 367 des Volllängen-NIP45-Proteins kodiert, worin die kodierten Proteine in der Lage sind, mit NFATp wechselzuwirken, um die Transkription des IL-4-Gens zu potenzieren.

**2.** Expressionsvektor, der ein beliebiges Polynucleotid nach Anspruch 1 enthält.

**3.** Wirtszelle, die einen Expressionsvektor nach Anspruch 2 enthält.

**4.** Im Wesentlichen gereinigtes Polypeptid, für das ein Polynucleotid nach Anspruch 1 kodiert.

**5.** Verfahren zur Produktion eines Polypeptids nach Anspruch 4, worin das Verfahren die folgenden Schritte umfasst:

a. das Kultivieren einer Wirtszelle nach Anspruch 3 unter Bedingungen, die für die Expression eines Polypeptids nach Anspruch 4 geeignet sind; und
b. das Gewinnen eines Polypeptids nach Anspruch 4 aus der Wirtszellenkultur.

**6.** Verfahren zur Detektion eines Polynucleotids nach Anspruch 1 in einer biologischen Probe, die einem menschlichen Körper oder Tierkörper entnommen wurde, umfassend die folgenden Schritte:

a. das Hybridisieren eines beliebigen Polynucleotids nach Anspruch 1 oder 2 an ein Nucleinsäurematerial einer biologischen Probe, die einem menschlichen Körper oder Tierkörper entnommen wurde, wodurch ein Hybridisierungskomplex gebildet wird; und
b. das Detektieren des Hybridisierungskomplexes.

**7.** Verfahren nach Anspruch 6, worin vor der Hybridisierung das Nucleinsäurematerial der biologischen Probe amplifiziert wird.

**8.** Verfahren zur Detektion eines Polypeptids nach Anspruch 4, umfassend die folgenden Schritte:

a. das Kontaktieren einer biologischen Probe, die einem menschlichen Körper oder Tierkörper entnommen wurde, mit einem Antikörper, der mit dem Polypeptid spezifisch wechselwirkt; und
b. das Detektieren der Wechselwirkung.

**9.** Diagnoseset zur Durchführung eines Verfahrens nach einem der Ansprüche 6 bis 8.

**10.** Verfahren zum Screenen auf Mittel, welche die Aktivität eines Polypeptids nach Anspruch 4 senken, umfassend die folgenden Schritte:

a. das Kontaktieren einer Testverbindung mit einem beliebigen Polypeptid, für das ein beliebiges Polynucleotid nach Anspruch 1 kodiert, oder mit einem beliebigen Polynucleotid nach Anspruch 1;
b. das Detektieren von Bindung der Testverbindung an das Polypeptid, für das ein beliebiges Polynucleotid nach Anspruch 1 kodiert, oder an das Polynucleotid nach Anspruch 1, wobei eine Testverbindung, die sich an das Polypeptid oder das Polynucleotid bindet, als potenzielles therapeutisches Mittel zur Senkung der Aktivität eines NIP45-Spleißvarianten-Polypeptids nach Anspruch 4 identifiziert wird.

**11.** Verfahren zum Screenen auf Mittel, welche die Aktivität eines Polypeptids nach Anspruch 4 regulieren, umfassend die folgenden Schritte:

a. das Kontaktieren einer Testverbindung mit einem Polypeptid, für das ein beliebiges Polynucleotid nach Anspruch 1 oder 2 kodiert; und
b. das Detektieren einer Aktivität des Polypeptids, wobei eine Testverbindung, welche die Aktivität steigert, als potenzielles therapeutisches Mittel zur Steigerung der Aktivität des Polypeptids identifiziert wird, und wobei eine Testverbindung, welche die Aktivität des Polypeptids senkt, als potenzielles therapeutisches Mittel zur Senkung der Aktivität des Polypeptids identifiziert wird.

**12.** Pharmazeutische Zusammensetzung, umfassend einen Expressionsvektor nach Anspruch 2 und einen pharmazeutisch annehmbaren Träger.

**13.** Verwendung eines Expressionsvektors nach Anspruch 2 bei der Herstellung eines Medikaments zur Modulierung der Aktivität eines NIP45-Spleißvarianten-Polypeptids, dem im Vergleich mit dem Volllängen-NIP45-Protein die Aminosäurereste 283 bis 367 fehlen, im Falle einer Erkrankung.

**14.** Verwendung nach Anspruch 13, worin die Erkrankung eine Autoimmunerkrankung, allergische, infektiöse oder chronische Entzündungserkrankung ist.

**15.** Verwendung nach Anspruch 14, worin die Erkrankung Asthma ist.

## Revendications

**1.** Polynucléotide isolé choisi dans le groupe consistant en

i) polynucléotides codant un polypeptide comprenant une séquence d'acides aminés représentée dans SEQ ID N° 8 manquant des résidus d'acides aminés 283 à 367 par rapport à la protéine NIP45 de longueur complète, qui sont capables d'interagir avec NFATp afin de potentialiser la transcription du gène de IL-4 ;
ii) polynucléotides comprenant la séquence de SEQ ID N° 2 manquant de la partie de séquence codant pour les résidus d'acides aminés 283 à 367 par rapport à la protéine NIP45 de longueur complète, les protéines codées étant capables d'interagir avec NFATp afin de potentialiser la transcription du gène de IL-4.

**2.** Vecteur d'expression contenant tout polynucléotide suivant la revendication 1.

**3.** Cellule hôte contenant le vecteur d'expression suivant la revendication 2.

**4.** Polypeptide sensiblement purifié codé par un polynucléotide suivant la revendication 1.

**5.** Procédé de production d'un polypeptide suivant la revendication 4, procédé comprenant les étapes suivantes; qui consistent :

a. à cultiver la cellule hôte suivant la revendication 3 dans des conditions qui conviennent pour l'expression du polypeptide suivant la revendication 4 ; et
b. à recueillir le polypeptide suivant la revendication 4 en l'isolant de la culture de la cellule hôte.

**6.** Méthode de détection d'un polynucléotide suivant la revendication 1 dans un échantillon biologique prélevé d'un corps humain ou animal, comprenant les étapes suivantes, qui consistent :

a. à hybrider tout polynucléotide suivant la revendication 1 ou 2 avec un acide nucléique présent dans un échantillon biologique prélevé d'un corps humain ou animal de manière à former ainsi un complexe d'hybridation ; et
b. à détecter le complexe d'hybridation ainsi formé.

**7.** Méthode suivant la revendication 6, dans laquelle l'acide nucléique de l'échantillon biologique est amplifié préalablement à l'hybridation.

**8.** Méthode de détection d'un polypeptide suivant la revendication 4, comprenant les étapes qui consistent :

a. à mettre en contact un échantillon biologique prélevé d'un corps humain ou animal avec un anticorps qui interagit spécifiquement avec le polypeptide, et
b. à détecter l'interaction.

**9.** Nécessaire de diagnostic destiné à la conduite de la méthode suivant l'une quelconque des revendications 6 à 8.

**10.** Méthode de triage pour la détection d'agents qui abaissent l'activité d'un polypeptide suivant la revendication 4, comprenant les étapes qui consistent :

a. à mettre un composé à tester en contact avec tout polypeptide codé par tout polynucléotide suivant la revendication 1 ou avec tout polynucléotide suivant la revendication 1 ;
b. à détecter la liaison du composé à tester au polypeptide codé par tout polynucléotide suivant la revendication 1 ou au polynucléotide suivant la revendication 1, un composé à tester qui se lie au polypeptide ou au polynucléotide étant identifié comme un agent thérapeutique potentiel susceptible d'abaisser l'activité d'un polypeptide variant d'épissage de NIP45 suivant la revendication 4.

**11.** Méthode de triage pour la détection d'agents régulateurs de l'activité d'un polypeptide suivant la revendication 4, comprenant les étapes qui consistent :

a. à mettre un composé à tester en contact avec un polypeptide codé par tout polynucléotide suivant la revendication 1 ou 2 ; et
b. à détecter une activité du polypeptide, un composé à tester qui élève l'activité étant identifié comme un agent thérapeutique potentiel susceptible d'élever l'activité du polypeptide, et un composé à tester qui abaisse l'activité du polypeptide étant identifié comme un agent thérapeutique potentiel susceptible d'abaisser l'activité du polypeptide.

**12.** Composition pharmaceutique, comprenant le vecteur d'expression suivant la revendication 2 et un support pharmaceutiquement acceptable.

**13.** Utilisation du vecteur d'expression suivant la revendication 2 dans la préparation d'un médicament destiné à moduler, dans une maladie, l'activité d'un polypeptide variant d'épissage de NIP45 privé des résidus d'acides aminés 283 à 367 par rapport à la protéine NIP45 de longueur complète.

**14.** Utilisation suivant la revendication 13, dans laquelle la maladie est une maladie auto-immune, une maladie allergique, une maladie infectieuse ou une maladie inflammatoire chronique.

**15.** Utilisation suivant la revendication 14, dans laquelle la maladie est l'asthme.

FIG. 1

ggaaagtgtg ccatggcgga gcctgtgggg aagcggggcc gctggtccgg aggtagcggt 60
gccggccgag ggggtcgggg cggctggggc ggtcggggcc gggctcctcg ggcccagcgg 120
tctccatccc ggggcacgct ggacgtagtg tctgtggact tggtcaccga cagcgatgag 180
gaaattctgg aggtcgccac cgctcgcggt gccgcggacg aggttgaggt ggagcccccg 240
gagcccccgg ggccggtcgc gtcccgggat aacagcaaca gtgacagcga aggggaggac 300
aggcggcccg caggaccccc gcgggagccg gtcaggcggc ggcggcggct ggtgctggat 360
ccgggggagg cgccgctggt tccggtgtac tcggggaagg ttaaaagcag ccttcgcctt 420
atcccagatg atctatccct cctgaaactc taccctccag ggatgagga agaggcagag 480
ctggcagatt cgagtggtct ctaccatgag ggctccccat caccaggctc tccctggaag 540
acaaagctga ggactaagga taaagaagag aagaaaaaga cagagtttct ggatctggac 600
aactctcctc tgtccccacc ttcaccaagg accaaaagca gaacgcatac tcgggcactc 660
aagaagttaa gtgaggtgaa caagcgcctc caggatctcc gttcctgtct gagccccaag 720
ccacctcagg gtcaagagca acagggccaa gaggatgaag tggtcttggt ggaagggccc 780
accctcccag agacccccg actcttccca ctcaaaatcc gttgccgggc tgacctggtc 840
agattgcccc tcaggatgga ttcccctcta aagaccctca tgtcccacta tgaggaggcc 900
atgggactgt cgggacggaa gctctccttc ttctttgatg ggacaaagct ttcaggcagg 960
gagctgccag ctgacctggg catggaatct ggggacctca ttgaggtctg ggctgacacc 1020
ccactccctg tttgacggcc cagcctggac ttggggagaa tgactttccc ttttttgccc 1080
cataagggct agcataagct gaggtagaac ttatctttaa gctgcagcaa aatcaaggag 1140
tgacttttgt cccctctcct gttgaccctg gtttagagcc gttaaccact tggtgagtta 1200
tgtgggtgtt gttgccctgg gtggcctgtg gctccgtcca caagtcatgc tgagttttgc 1260
agcctctgtg acttggagat gtcccttcac ccctcccctt tcaccaccat cctcttttcc 1320
tcatggaaat gtctgcttta tgaaactatg cacatattga aagtgagttg aaacaaatga 1380
gggttgggta ggagcttcca ggcctgggat ttacaccacg cctagcccag cagaggcctt 1440
agtcccattt ggggcttggg agtgacattt gcttgaggct tatacactgg tgtggttgcc 1500
tggcttgcag gaaatgacca agctcacaca tgctggctga agcgtaagca gacaactgag 1560
gtactctttt gaaggatgaa ggtggtggat tctcagccct gggggtcttc ctcacctgag 1620
gacccttcag agccaccctt tctagtttgc atttcctggt gcacacattt aaggcataac 1680
agcacattca tccctttggt ttgggatctc aggaatacag tcccatgcaa agattctctg 1740

```
gttttatggc ttttttccct ttctttacac catcctctcc cataagcacc catgtctttg 1800
aatatgaatg tatttgtaaa ataccacgtt tcatgtgtga atatgtgctt ttactgtaca 1860
tagtgctatt gtgcaatagg tcttatgctg ttttcactca atgtgtgcta agatctagcc 1920
ccattgactc ttctagaaat gcagtattgc tttgacctgc catgtggcac tccacaatgt 1980
caattgcagt ttacacacat tgcctaaagt gggggacacc tgggtgcccc tgaccccttg 2040
gcaccggata caggccacga taaacatcct ttcgtgtgtt cccttctgtg cttgtgtggc 2100
atgtgtaccc aggatgggcc tataggtcac agaggtcagt ttctctttgg ttttccagat 2160
tttctttaga acggtgactg accctcctac ttgaggccgc ccttttctcc ttatccttgc 2220
cagcacttgt at                                                     2232
```

Fig. 2

```
>NIP45v1_nt 255 bp deletion; 1005 bp
ATGGCGGAGCCTGTGGGGAAGCGGGGCCGCTGGTCCGGAGGTAGCGGTGCCGGCCGAGGGGGTCGGGGCGGCTGGGGCGGTCGG
GGCCGGGCTCCTCGGGCCCAGCGGTCTCCATCCCGGGGCACGCTGGACGTAGTGTCTGTGGACTTGGTCACCGACAGCGATGAG
GAAATTCTGGAGGTCGCCACCGCTCGCGGTGCCGCGGACGAGGTTGAGGTGGAGCCCCCGGAGCCCCCGGGGCCGGTCGCGTCC
CGGGATAACAGCAACAGTGACAGCGAAGGGGAGGACAGGCGGCCCGCAGGACCCCCGCGGGAGCCGGTCAGGCGGCGGCGGCGG
CTGGTGCTGGATCCGGGGGAGGCGCCGCTGGTTCCGGTGTACTCGGGGAAGGTTAAAAGCAGCCTTCGCCTTATCCCAGATGAT
CTATCCCTCCTGAAACTCTACCCTCCAGGGGATGAGGAAGAGGCAGAGCTGGCAGATTCGAGTGGTCTCTACCATGAGGGCTCC
CCATCACCAGGCTCTCCCTGGAAGACAAAGCTGAGGACTAAGGATAAAGAAGAGAAGAAAAAGACAGAGTTTCTGGATCTGGAC
AACTCTCCTCTGTCCCCACCTTCACCAAGGACCAAAAGCAGAACGCATACTCGGGCACTCAAGAAGTTAAGTGAGGTGAACAAG
CGCCTCCAGGATCTCCGTTCCTGTCTGAGCCCCAAGCCACCTCAGGGTCAAGAGCAACAGGGCCAAGAGGATGAAGTGGTCTTG
GTGGAAGGGCCCACCCTCCCAGAGACCCCCCGACTCTTCCCACTCAAAATCCGTTGCCGGGCTGACCTGGTCAGATTGCCCCTC
AGGATGGATTCCCCTCTAAAGACCCTCATGTCCCACTATGAGGAGGCCATGGACTGTCGGGACGGAAGCTCTCCTTCTTCTTT
GATGGGACAAAGCTTTCAGGCAGGGAGCTGCCAGCTGACCTGGGCATGGAATCTGGGGACCTCATTGAGGTCTGGGGCTGA
```

Fig. 3

```
>NIP45v2_nt 145 bp deletion; 1115 bp
ATGGCGGAGCCTGTGGGGAAGCGGGGCCGCTGGTCCGGAGGTAGCGGTGCCGGCCGAGGGGGTCGGGGCGGCTGGGGCGGTCGG
GGCCGGGCTCCTCGGGCCCAGCGGTCTCCATCCCGGGGCACGCTGGACGTAGTGTCTGTGGACTTGGTCACCGACAGCGATGAG
GAAATTCTGGAGGTCGCCACCGCTCGCGGTGCCGCGGACGAGGTTGAGGTGGAGCCCCCGGAGCCCCCGGGGCCGGTCGCGTCC
CGGGATAACAGCAACAGTGACAGCGAAGGGGAGGACAGGCGGCCCGCAGGACCCCCGCGGGAGCCGGTCAGGCGGCGGCGGCGG
CTGGTGCTGGATCCGGGGGAGGCGCCGCTGGTTCCGGTGTACTCGGGGAAGGTTAAAAGCAGCCTTCGCCTTATCCCAGATGAT
CTATCCCTCCTGAAACTCTACCCTCCAGGGGATGAGGAAGAGGCAGAGCTGGCAGATTCGAGTGGTCTCTACCATGAGGGCTCC
CCATCACCAGGCTCTCCCTGGAAGACAAAGCTGAGGACTAAGGATAAAGAAGAGAAGAAAAAGACAGAGTTTCTGGATCTGGAC
AACTCTCCTCTGTCCCCACCTTCACCAAGGACCAAAAGCAGAACGCATACTCGGGCACTCAAGAAGTTAAGTGAGGTGAACAAG
CGCCTCCAGGATCTCCGTTCCTGTCTGAGCCCCAAGCCACCTCAGGGTCAAGAGCAACAGGGCCAAGAGGATGAAGTGGTCTTG
GTGGAAGGGCCCACCCTCCCAGAGACCCCCCGACTCTTCCCACTCAAAATCCGTTGCCGGGCTGACCTGGTCAGATTGCCCCTC
AGGATGACTGTGTGGTACTAACAAGTTCTCCAGAGGCCACAGAGACGTCCCAACAGCTCCAGCTCCGGGTGCAGGGAAAGGAGA
AACACCAGACACTGGAAGTCTCACTGTCTCGAGATTCCCCTCTAAAGACCCTCATGTCCCACTATGAGGAGGCCATGGACTGT
CGGGACGGAAGCTCTCCTTCTTCTTTGATGGGACAAAGCTTTCAGGCAGGGAGCTGCCAGCTGACCTGGGCATGGAATCTGGGG
ACCTCATTGAGGTCTGGGGCTGA
```

Fig. 4

```
>NIP45v3_nt 347 bp deletion; 913 bp
ATGGCGGAGCCTGTGGGGAAGCGGGGCCGCTGGTCCGGAGGTTAAAAGCAGCCTTCGCCTTATCCCAGATGATCTATCCCTCCT
GAAACTCTACCCTCCAGGGGATGAGGAAGAGGCAGAGCTGGCAGATTCGAGTGGTCTCTACCATGAGGGCTCCCCATCACCAGG
CTCTCCCTGGAAGACAAAGCTGAGGACTAAGGATAAAGAAGAGAAGAAAAAGACAGAGTTTCTGGATCTGGACAACTCTCCTCT
GTCCCCACCTTCACCAAGGACCAAAAGCAGAACGCATACTCGGGCACTCAAGAAGTTAAGTGAGGTGAACAAGCGCCTCCAGGA
TCTCCGTTCCTGTCTGAGCCCCAAGCCACCTCAGGGTCAAGAGCAACAGGGCCAAGAGGATGAAGTGGTCTTGGTGGAAGGGCC
CACCCTCCCAGAGACCCCCCGACTCTTCCCACTCAAAATCCGTTGCCGGGCTGACCTGGTCAGATTGCCCCTCAGGATGTCGGA
GCCCCTGCAGAGTGTGGTGGACCACATGGCCACCCACCTTGGGGTGTCCCCAAGCAGGATCCTTTTGCTTTTTGGAGAGACAGA
GCTATCACCTACTGCCACTCCCAGGACCCTAAAGCTCGGAGTGGCTGACATCATTGACTGTGTGGTACTAACAAGTTCTCCAGA
GGCCACAGAGACGTCCCAACAGCTCCAGCTCCGGGTGCAGGGAAAGGAGAAACACCAGACACTGGAAGTCTCACTGTCTCGAGA
TTCCCCTCTAAAGACCCTCATGTCCCACTATGAGGAGGCCATGGGACTGTCGGGACGGAAGCTCTCCTTCTTCTTTGATGGGAC
AAAGCTTTCAGGCAGGGAGCTGCCAGCTGACCTGGCATGGAATCTGGGGACCTCATTGAGGTCTGGGGCTGA
```

Fig. 5

```
>NIP45v4_nt 988 bp deletion; 272 bp
ATGGCGGAGCCTGTGGGGAAGCGGGGCCGCTGGTCCGGAGGTTAAAAGCAGCCTTCGCCTTATCCCAGATGATCTATCCCTCCT
GAAACTCTACCCTCCAGGGGATGAGGAAGGATTCCCCTCTAAAGACCCTCATGTCCCACTATGAGGAGGCCATGGGACTGTCGG
GACGGAAGCTCTCCTTCTTCTTTGATGGGACAAAGCTTTCAGGCAGGGAGCTGCCAGCTGACCTGGCATGGAATCTGGGGACC
TCATTGAGGTCTGGGGCTGA
```

Fig. 6

```
ggaaagtgtg ccatggcgga gcctgtgggg aagcggggcc gctggtccgg aggtagcggt   60
gccggccgag gggtcgggg cggctggggc ggtcggggcc gggctcctcg ggcccagcgg  120
tctccatccc ggggcacgct ggacgtagtg tctgtggact tggtcaccga cagcgatgag  180
gaaattctgg aggtcgccac cgctcgcggt gccgcggacg aggttgaggt ggagcccccg  240
gagcccccgg ggccggtcgc gtcccgggat aacagcaaca gtgacagcga aggggaggac  300
aggcggcccg caggaccccc gcgggagccg gtcaggcggc ggcggcggct ggtgctggat  360
ccgggggagg cgccgctggt tccggtgtac tcggggaagg ttaaaagcag ccttcgcctt  420
atcccagatg atctatccct cctgaaactc taccctccag gggatgagga agaggcagag  480
ctggcagatt cgagtggtct ctaccatgag ggctccccat caccaggctc tccctggaag  540
acaaagctga ggactaagga taaagaagag aagaaaaaga cagagtttct ggatctggac  600
aactctcctc tgtccccacc ttcaccaagg accaaaagca gaacgcatac tcgggcactc  660
aagaagttaa gtgaggtgaa caagcgcctc caggatctcc gttcctgtct gagccccaag  720
ccacctcagg gtcaagagca acagggccaa gaggatgaag tggtcttggt ggaagggccc  780
```

accctcccag agaccccccg actcttccca ctcaaaatcc gttgccgggc tgacctggtc 840

agattgcccc tcaggatgtc ggagcccctg cagagtgtgg tggaccacat ggccacccac 900

cttggggtgt ccccaagcag gatccttttg ctttttggag agacagagct atcacctact 960

gccactccca ggaccctaaa gctcggagtg gctgacatca ttgactgtgt ggtactaaca 1020

agttctccag aggccacaga gacgtcccaa cagctccagc tccgggtgca gggaaaggag 1080

aaacaccaga cactggaagt ctcactgtct cgagattccc ctctaaagac cctcatgtcc 1140

cactatgagg aggccatggg actgtcggga cggaagctct ccttcttctt tgatgggaca 1200

aagctttcag gcagggagct gccagctgac ctgggcatgg aatctgggga cctcattgag 1260

gtctggggct gacaccccac tccctgtttg acggcccagc ctggacttgg ggagaatgac 1320

tttccctttt ttgccccata agggctagca taagctgagg tagaacttat ctttaagctg 1380

cagcaaaatc aaggagtgac ttttgtcccc tctcctgttg accctggttt agagccgtta 1440

accacttggt gagttatgtg ggtgttgttg ccctgggtgg cctgtggctc cgtccacaag 1500

tcatgctgag ttttgcagcc tctgtgactt ggagatgtcc cttcacccct cccctttcac 1560

caccatcctc ttttcctcat ggaaatgtct gctttatgaa actatgcaca tattgaaagt 1620

gagttgaaac aaatgagggt tgggtaggag cttccaggcc tgggatttac accacgccta 1680

gcccagcaga ggccttagtc ccatttgggg cttgggagtg acatttgctt gaggcttata 1740

cactggtgtg gttgcctggc ttgcaggaaa tgaccaagct cacacatgct ggctgaagcg 1800

taagcagaca actgaggtac tcttttgaag gatgaaggtg gtggattctc agccctgggg 1860

gtcttcctca cctgaggacc cttcagagcc accctttcta gtttgcattt cctggtgcac 1920

acatttaagg cataacagca cattcatccc tttggtttgg gatctcagga atacagtccc 1980

atgcaaagat tctctggttt tatggctttt ttccctttct ttacaccatc ctctcccata 2040

agcacccatg tctttgaata tgaatgtatt tgtaaaatac cacgtttcat gtgtgaatat 2100

gtgcttttac tgtacatagt gctattgtgc aataggtctt atgctgtttt cactcaatgt 2160

gtgctaagat ctagccccat tgactcttct agaaatgcag tattgctttg acctgccatg 2220

tggcactcca caatgtcaat tgcagtttac acacattgcc taaagtgggg gacacctggg 2280

tgcccctgac cccttggcac cggatacagg ccacgataaa catcctttcg tgtgttccct 2340

tctgtgcttg tgtggcatgt gtacccagga tgggcctata ggtcacagag gtcagtttct 2400

ctttggtttt ccagattttc tttagaacgg tgactgaccc tcctacttga ggccgccctt 2460

ttctccttat ccttgccagc acttgtattg ccagactacc taatttttgc cagtctcatg 2520

ggtagatagt ggtgcagtgc tttaacatac attcatctga tcagcattaa tttggg 2576

Fig. 7

>NIP45_nt full length; 1260 bp
ATGGCGGAGCCTGTGGGGAAGCGGGGCCGCTGGTCCGGAGGTAGCGGTGCCGGCCGAGGGGGTCGGGGCGGCTGGGGCGGTCGG
GGCCGGGCTCCTCGGGCCCAGCGGTCTCCATCCCGGGGCACGCTGGACGTAGTGTCTGTGGACTTGGTCACCGACAGCGATGAG
GAAATTCTGGAGGTCGCCACCGCTCGCGGTGCCGCGGACGAGGTTGAGGTGGAGCCCCCGGAGCCCCCGGGGCCGGTCGCGTCC
CGGGATAACAGCAACAGTGACAGCGAAGGGGAGGACAGGCGGCCCGCAGGACCCCCGCGGGAGCCGGTCAGGCGGCGGCGGCGG
CTGGTGCTGGATCCGGGGGAGGCGCCGCTGGTTCCGGTGTACTCGGGGAAGGTTAAAAGCAGCCTTCGCCTTATCCCAGATGAT
CTATCCCTCCTGAAACTCTACCCTCCAGGGGATGAGGAAGAGGCAGAGCTGGCAGATTCGAGTGGTCTCTACCATGAGGGCTCC
CCATCACCAGGCTCTCCCTGGAAGACAAAGCTGAGGACTAAGGATAAAGAAGAGAAGAAAAAGACAGAGTTTCTGGATCTGGAC
AACTCTCCTCTGTCCCCACCTTCACCAAGGACCAAAAGCAGAACGCATACTCGGGCACTCAAGAAGTTAAGTGAGGTGAACAAG
CGCCTCCAGGATCTCCGTTCCTGTCTGAGCCCCAAGCCACCTCAGGGTCAAGAGCAACAGGGCCAAGAGGATGAAGTGGTCTTG
GTGGAAGGGCCCACCCTCCCAGAGACCCCCCGACTCTTCCCACTCAAAATCCGTTGCCGGGCTGACCTGGTCAGATTGCCCCTC
AGGATGTCGGAGCCCCTGCAGAGTGTGGTGGACCACATGGCCACCCACCTTGGGGTGTCCCCAAGCAGGATCCTTTTGCTTTTT
GGAGAGACAGAGCTATCACCTACTGCCACTCCCAGGACCCTAAAGCTCGGAGTGGCTGACATCATTGACTGTGTGGTACTAACA
AGTTCTCCAGAGGCCACAGAGACGTCCCAACAGCTCCAGCTCCGGGTGCAGGGAAAGGAGAAACACCAGACACTGGAAGTCTCA
CTGTCTCGAGATTCCCCTCTAAAGACCCTCATGTCCCACTATGAGGAGGCCATGGGACTGTCGGGACGGAAGCTCTCCTTCTTC
TTTGATGGGACAAAGCTTTCAGGCAGGGAGCTGCCAGCTGACCTGGGCATGGAATCTGGGGACCTCATTGAGGTCTGGGGCTGA

Fig. 8

>NIP45v1_aa 335 aa (three-letter code)
METAlaGluProValGlyLysArgGlyArgTrpSerGlyGlySerGlyAlaGlyArgGlyGlyArgGlyGlyTrpGlyGlyArg
GlyArgAlaProArgAlaGlnArgSerProSerArgGlyThrLeuAspValValSerValAspLeuValThrAspSerAspGlu
GluIleLeuGluValAlaThrAlaArgGlyAlaAlaAspGluValGluValGluProProGluProProGlyProValAlaSer
ArgAspAsnSerAsnSerAspSerGluGlyGluAspArgArgProAlaGlyProProArgGluProValArgArgArgArgArg
LeuValLeuAspProGlyGluAlaProLeuValProValTyrSerGlyLysValLysSerSerLeuArgLeuIleProAspAsp
LeuSerLeuLeuLysLeuTyrProProGlyAspGluGluGluAlaGluLeuAlaAspSerSerGlyLeuTyrHisGluGlySer
ProSerProGlySerProTrpLysThrLysLeuArgThrLysAspLysGluGluLysLysLysThrGluPheLeuAspLeuAsp
AsnSerProLeuSerProProSerProArgThrLysSerArgThrHisThrArgAlaLeuLysLysLeuSerGluValAsnLys
ArgLeuGlnAspLeuArgSerCysLeuSerProLysProProGlnGlyGlnGluGlnGlnGlyGlnGluAspGluValValLeu
ValGluGlyProThrLeuProGluThrProArgLeuPheProLeuLysIleArgCysArgAlaAspLeuValArgLeuProLeu
ArgMETAspSerProLeuLysThrLeuMETSerHisTyrGluGluAlaMETGlyLeuSerGlyArgLysLeuSerPhePhePhe
AspGlyThrLysLeuSerGlyArgGluLeuProAlaAspLeuGlyMETGluSerGlyAspLeuIleGluValTrpGlySTP

Fig. 9

>NIP45v2_aa 287 aa (three-letter code)
METAlaGluProValGlyLysArgGlyArgTrpSerGlyGlySerGlyAlaGlyArgGlyGlyArgGlyGlyTrpGlyGlyArg
GlyArgAlaProArgAlaGlnArgSerProSerArgGlyThrLeuAspValValSerValAspLeuValThrAspSerAspGlu
GluIleLeuGluValAlaThrAlaArgGlyAlaAlaAspGluValGluValGluProProGluProProGlyProValAlaSer
ArgAspAsnSerAsnSerAspSerGluGlyGluAspArgArgProAlaGlyProProArgGluProValArgArgArgArgArg
LeuValLeuAspProGlyGluAlaProLeuValProValTyrSerGlyLysValLysSerSerLeuArgLeuIleProAspAsp
LeuSerLeuLeuLysLeuTyrProProGlyAspGluGluGluAlaGluLeuAlaAspSerSerGlyLeuTyrHisGluGlySer
ProSerProGlySerProTrpLysThrLysLeuArgThrLysAspLysGluGluLysLysLysThrGluPheLeuAspLeuAsp
AsnSerProLeuSerProProSerProArgThrLysSerArgThrHisThrArgAlaLeuLysLysLeuSerGluValAsnLys
ArgLeuGlnAspLeuArgSerCysLeuSerProLysProProGlnGlyGlnGluGlnGlnGlyGlnGluAspGluValValLeu
ValGluGlyProThrLeuProGluThrProArgLeuPheProLeuLysIleArgCysArgAlaAspLeuValArgLeuProLeu
ArgMETThrValTrpTyrSTP

Fig. 10

>NIP45v3_aa 304 aa (three-letter code)
TrpArgSerLeuTrpGlySerGlyAlaAlaGlyProGluValLysSerSerLeuArgLeuIleProAspAspLeuSerLeuLeu
LysLeuTyrProProGlyAspGluGluGluAlaGluLeuAlaAspSerSerGlyLeuTyrHisGluGlySerProSerProGly
SerProTrpLysThrLysLeuArgThrLysAspLysGluGluLysLysLysThrGluPheLeuAspLeuAspAsnSerProLeu
SerProProSerProArgThrLysSerArgThrHisThrArgAlaLeuLysLysLeuSerGluValAsnLysArgLeuGlnAsp
LeuArgSerCysLeuSerProLysProProGlnGlyGlnGluGlnGlnGlyGlnGluAspGluValValLeuValGluGlyPro
ThrLeuProGluThrProArgLeuPheProLeuLysIleArgCysArgAlaAspLeuValArgLeuProLeuArgMETSerGlu
ProLeuGlnSerValValAspHisMETAlaThrHisLeuGlyValSerProSerArgIleLeuLeuLeuPheGlyGluThrGlu
LeuSerProThrAlaThrProArgThrLeuLysLeuGlyValAlaAspIleIleAspCysValValLeuThrSerSerProGlu
AlaThrGluThrSerGlnGlnLeuGlnLeuArgValGlnGlyLysGluLysHisGlnThrLeuGluValSerLeuSerArgAsp
SerProLeuLysThrLeuMETSerHisTyrGluGluAlaMETGlyLeuSerGlyArgLysLeuSerPhePhePheAspGlyThr
LysLeuSerGlyArgGluLeuProAlaAspLeuGlyMETGluSerGlyAspLeuIleGluValTrpGlySTP

Fig. 11

>NIP45v4_aa 55 aa (three-letter code)
METArgLysAspSerProLeuLysThrLeuMETSerHisTyrGluGluAlaMETGlyLeuSerGlyArgLysLeuSerPhePhe
PheAspGlyThrLysLeuSerGlyArgGluLeuProAlaAspLeuGlyMETGluSerGlyAspLeuIleGluValTrpGlySTP

Fig. 12

>NIP45_aa full length; 420 aa (three-letter code)

METAlaGluProValGlyLysArgGlyArgTrpSerGlyGlySerGlyAlaGlyArgGlyGlyArgGlyGlyTrpGlyGlyArg
GlyArgAlaProArgAlaGlnArgSerProSerArgGlyThrLeuAspValValSerValAspLeuValThrAspSerAspGlu
GluIleLeuGluValAlaThrAlaArgGlyAlaAlaAspGluValGluValGluProProGluProProGlyProValAlaSer
ArgAspAsnSerAsnSerAspSerGluGlyGluAspArgArgProAlaGlyProProArgGluProValArgArgArgArgArg
LeuValLeuAspProGlyGluAlaProLeuValProValTyrSerGlyLysValLysSerSerLeuArgLeuIleProAspAsp
LeuSerLeuLeuLysLeuTyrProProGlyAspGluGluGluAlaGluLeuAlaAspSerSerGlyLeuTyrHisGluGlySer
ProSerProGlySerProTrpLysThrLysLeuArgThrLysAspLysGluGluLysLysLysThrGluPheLeuAspLeuAsp
AsnSerProLeuSerProProSerProArgThrLysSerArgThrHisThrArgAlaLeuLysLysLeuSerGluValAsnLys
ArgLeuGlnAspLeuArgSerCysLeuSerProLysProProGlnGlyGlnGluGlnGlnGlyGlnGluAspGluValValLeu
ValGluGlyProThrLeuProGluThrProArgLeuPheProLeuLysIleArgCysArgAlaAspLeuValArgLeuProLeu
ArgMETSerGluProLeuGlnSerValValAspHisMETAlaThrHisLeuGlyValSerProSerArgIleLeuLeuLeuPhe
GlyGluThrGluLeuSerProThrAlaThrProArgThrLeuLysLeuGlyValAlaAspIleIleAspCysValValLeuThr
SerSerProGluAlaThrGluThrSerGlnGlnLeuGlnLeuArgValGlnGlyLysGluLysHisGlnThrLeuGluValSer
LeuSerArgAspSerProLeuLysThrLeuMETSerHisTyrGluGluAlaMETGlyLeuSerGlyArgLysLeuSerPhePhe
PheAspGlyThrLysLeuSerGlyArgGluLeuProAlaAspLeuGlyMETGluSerGlyAspLeuIleGluValTrpGlySTP

Fig. 13

PCR amplification of human NIP45

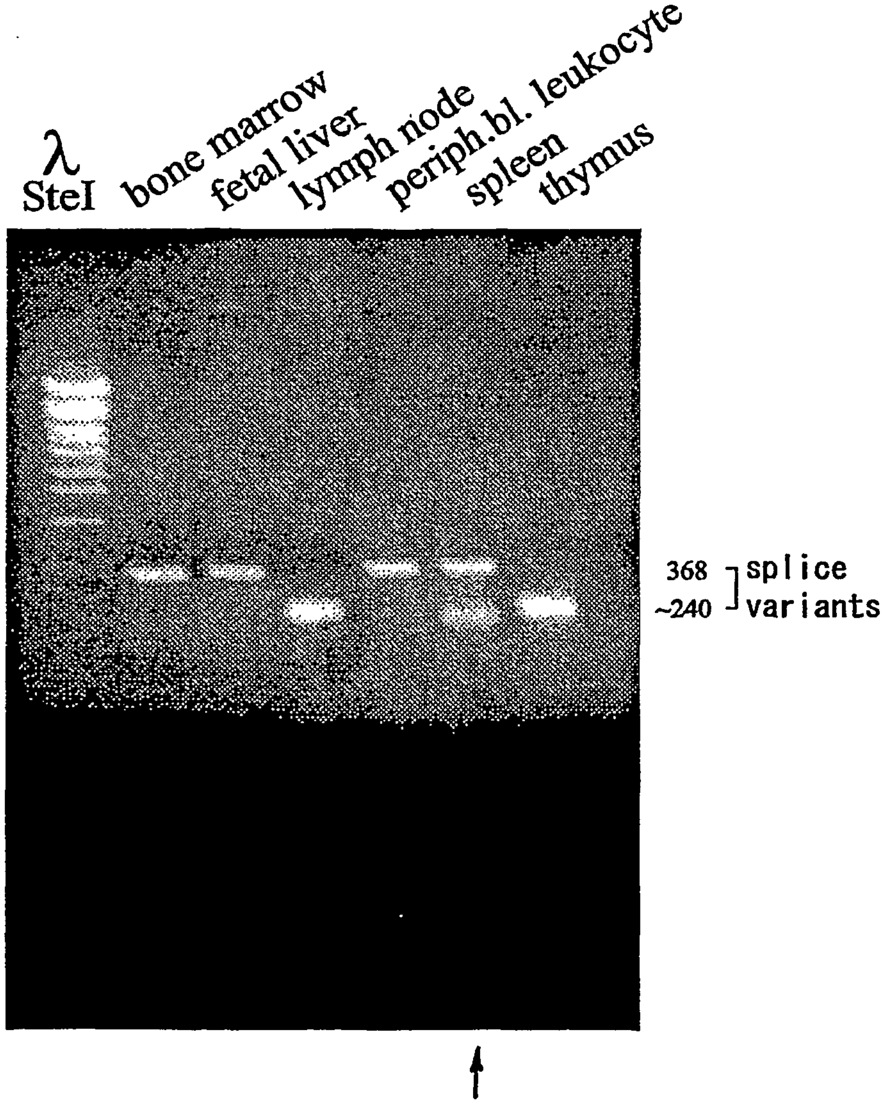

Fig. 14

| | | |
|---|---|---|
| | | Section 1 |
| | (1)1 10 20 30 40 51 | |
| NIP45_aa | (1)MAEPVGKRGRWSGGSGAGRGGRGGWGGRGRAPRAQRSPSRGTLDVVSVDLV | |
| NIP45v1_aa | (1)MAEPVGKRGRWSGGSGAGRGGRGGWGGRGRAPRAQRSPSRGTLDVVSVDLV | |
| NIP45v2_aa | (1)MAEPVGKRGRWSGGSGAGRGGRGGWGGRGRAPRAQRSPSRGTLDVVSVDLV | |
| NIP45v3_aa | (1)--------------------------------------------------- | |
| NIP45v4_aa | (1)--------------------------------------------------- | |
| Consensus | (1)MAEPVGKRGRWSGGSGAGRGGRGGWGGRGRAPRAQRSPSRGTLDVVSVDLV | |
| | | Section 2 |
| | (52)52 60 70 80 90 102 | |
| NIP45_aa | (52)TDSDEEILEVATARGAADEVEVEPPEPPGPVASRDNSNSDSEGEDRRPAGP | |
| NIP45v1_aa | (52)TDSDEEILEVATARGAADEVEVEPPEPPGPVASRDNSNSDSEGEDRRPAGP | |
| NIP45v2_aa | (52)TDSDEEILEVATARGAADEVEVEPPEPPGPVASRDNSNSDSEGEDRRPAGP | |
| NIP45v3_aa | (1) --------------------------------------------------- | |
| NIP45v4_aa | (1) --------------------------------------------------- | |
| Consensus | (52)TDSDEEILEVATARGAADEVEVEPPEPPGPVASRDNSNSDSEGEDRRPAGP | |

```
------------------------------------------------------------------------ --Section 3
               (103)103   110        120        130        140         153
NIP45_aa       (103)PREPVRRRRRLVLDPGEAPLVPVYSGKVKSSLRLIPDDLSLLKLYPPGDEE
NIP45v1_aa     (103)PREPVRRRRRLVLDPGEAPLVPVYSGKVKSSLRLIPDDLSLLKLYPPGDEE
NIP45v2_aa     (103)PREPVRRRRRLVLDPGEAPLVPVYSGKVKSSLRLIPDDLSLLKLYPPGDEE
NIP45v3_aa     (1)  --------------WRSLWGSGAAGPEVKSSLRLIPDDLSLLKLYPPGDEE
NIP45v4_aa     (1)  ---------------------------------------------------
Consensus      (103)PREPVRRRRRLVLDPGEAPLVPVYSGKVKSSLRLIPDDLSLLKLYPPGDEE
------------------------------------------------------------------------ --Section 4
               (154)154  160        170        180         190         204
NIP45_aa       (154)EAELADSSGLYHEGSPSPGSPWKTKLRTKDKEEKKKTEFLDLDNSPLSPPS
NIP45v1_aa     (154)EAELADSSGLYHEGSPSPGSPWKTKLRTKDKEEKKKTEFLDLDNSPLSPPS
NIP45v2_aa     (154)EAELADSSGLYHEGSPSPGSPWKTKLRTKDKEEKKKTEFLDLDNSPLSPPS
NIP45v3_aa     (38) EAELADSSGLYHEGSPSPGSPWKTKLRTKDKEEKKKTEFLDLDNSPLSPPS
NIP45v4_aa     (1)  ---------------------------------------------------
Consensus      (154)EAELADSSGLYHEGSPSPGSPWKTKLRTKDKEEKKKTEFLDLDNSPLSPPS
```

```
---------------------------------------------------------------------Section 5
              (205)205  210        220        230        240          255
NIP45_aa      (205)PRTKSRTHTRALKKLSEVNKRLQDLRSCLSPKPPQGQEQQGQEDEVVLVEG
NIP45v1_aa    (205)PRTKSRTHTRALKKLSEVNKRLQDLRSCLSPKPPQGQEQQGQEDEVVLVEG
NIP45v2_aa    (205)PRTKSRTHTRALKKLSEVNKRLQDLRSCLSPKPPQGQEQQGQEDEVVLVEG
NIP45v3_aa    (89) PRTKSRTHTRALKKLSEVNKRLQDLRSCLSPKPPQGQEQQGQEDEVVLVEG
NIP45v4_aa    (1)  ---------------------------------------------------
Consensus     (205)PRTKSRTHTRALKKLSEVNKRLQDLRSCLSPKPPQGQEQQGQEDEVVLVEG

---------------------------------------------------------------------Section 6
              (256)256 260        270        280        290           306
NIP45_aa      (256)PTLPETPRLFPLKIRCRADLVRLPLRMSEPLQSVVDHMATHLGVSPSRILL
NIP45v1_aa    (256)PTLPETPRLFPLKIRCRADLVRLPLRM------------------------
NIP45v2_aa    (256)PTLPETPRLFPLKIRCRADLVRLPLRMTVWY--------------------
NIP45v3_aa    (140)PTLPETPRLFPLKIRCRADLVRLPLRMSEPLQSVVDHMATHLGVSPSRILL
NIP45v4_aa    (1)  ---------------------------------------------------
Consensus     (256)PTLPETPRLFPLKIRCRADLVRLPLRMS
```

Section 7

| | | |
|---|---|---|
| | (307)307 | 320 330 340 357 |
| NIP45_aa | (307) | LFGETELSPTATPRTLKLGVADIIDCVVLTSSPEATETSQQLQLRVQGKEK |
| NIP45v1_aa | (283) | --------------------------------------------------- |
| NIP45v2_aa | (287) | --------------------------------------------------- |
| NIP45v3_aa | (191) | LFGETELSPTATPRTLKLGVADIIDCVVLTSSPEATETSQQLQLRVQGKEK |
| NIP45v4_aa | (1) | --------------------------------------------------- |
| Consensus | (307) | |

Section 8

| | | |
|---|---|---|
| | (358)358 | 370 380 390 408 |
| NIP45_aa | (358) | HQTLEVSLSRDSPLKTLMSHYEEAMGLSGRKLSFFFDGTKLSGRELPADLG |
| NIP45v1_aa | (283) | ----------DSPLKTLMSHYEEAMGLSGRKLSFFFDGTKLSGRELPADLG |
| NIP45v2_aa | (287) | --------------------------------------------------- |
| NIP45v3_aa | (242) | HQTLEVSLSRDSPLKTLMSHYEEAMGLSGRKLSFFFDGTKLSGRELPADLG |
| NIP45v4_aa | (1) | -------MRKDSPLKTLMSHYEEAMGLSGRKLSFFFDGTKLSGRELPADLG |
| Consensus | (358) | L RDSPLKTLMSHYEEAMGLSGRKLSFFFDGTKLSGRELPADLG |

-------------------------------------------------------------------------------Section 9

| | | |
|---|---|---|
| | (409)409 | 419 |
| NIP45_aa | (409)MESGDLIEVWG | |
| NIP45v1_aa | (324)MESGDLIEVWG | |
| NIP45v2_aa | (287)----------- | |
| NIP45v3_aa | (293)MESGDLIEVWG | |
| NIP45v4_aa | (45) MESGDLIEVWG | |
| Consensus | (409)MESGDLIEVWG | |